# EUROPEAN PATENT SPECIFICATION

(11) **EP 4 196 791 B1**
(45) Date of publication and mention of the grant of the patent: **30.04.2025**
(21) Application number: 21762077.2
(22) Date of filing: 13.08.2021
(51) Int. Cl.: G01N 33/574

(54) **METASTATIC BIOMARKER**
METASTATISCHER BIOMARKER
BIOMARQUEUR MÉTASTATIQUE

(30) Priority: 14.08.2020 GB 202012760
(43) Date of publication of application: 21.06.2023
(73) Proprietor: Oxford Brookes University, Oxfordshire OX3 0BP (GB)
(72) Inventor: PINK, Ryan, Oxford Oxfordshire OX3 0BP (GB); BROOKS, Susan, Oxford Oxfordshire OX3 0BP (GB); CARTER, David, Oxford Oxfordshire OX3 0BP (GB); BEAMAN, Ellie, Oxford Oxfordshire OX3 0BP (GB)
(74) Representative: Barker Brettell LLP
(86) International application number: PCT/GB2021/052110
(87) International publication number: WO 2022/034343

(56) References cited:
- EP-A1- 2 998 406
- WO-A2-2013/134786
- CN-A- 106 526 185
- ANDRONICOS NM ET AL: "The topology of plasminogen binding and activation on the surface of human breast cancer cells", BRITISH JOURNAL OF CANCER, 1 January 2001 (2001-01-01), XP055956528, Retrieved from the Internet <URL:https://www.ncbi.nlm.nih.gov/pmc/articles/PMC2375062/pdf/85-6692022a.pdf> [retrieved on 20220831], DOI: 10.1054/ bjoc.2001.2022
- H D SOULE ET AL: "A Human Cell Line From a Pleural Effusion Derived From a Breast Carcinoma", JOURNAL OF THE NATIONAL CANCER INSTITUTE, vol. 51, no. 5, 1 November 1973 (1973-11-01), pages 1409 - 1416, XP055634632, DOI: 10.1093/jnci/51.5.1409
- KATRIN STBKE ET AL: "Selectin-deficiency reduces the number of spontaneous metastases in a xenograft model of human breast cancer", CANCER LETTERS, NEW YORK, NY, US, vol. 321, no. 1, 16 February 2012 (2012-02-16), pages 89 - 99, XP028420273, ISSN: 0304-3835, [retrieved on 20120223], DOI: 10.1016/J.CANLET.2012.02.019
- SUDAKOV N P ET AL: "Extracellular actin in health and disease", BIOCHEMISTRY, MAIK NAUKA - INTERPERIODICA, RU, vol. 82, no. 1, 20 January 2017 (2017-01-20), pages 1 - 12, XP036140174, ISSN: 0006-2979, [retrieved on 20170120], DOI: 10.1134/S0006297917010011
- WANG HAO ET AL: "Cell Surface-Dependent Generation of Angiostatin4.5", CANCER RESEARCH, vol. 64, no. 1, 1 January 2004 (2004-01-01), US, pages 162 - 168, XP055848985, ISSN: 0008-5472, Retrieved from the Internet <URL:https://cancerres.aacrjournals.org/content/canres/64/1/162.full.pdf> DOI: 10.1158/0008-5472.CAN-03-1862
- MILES L. A. ET AL: "Cell-Surface Actin Binds Plasminogen and Modulates Neurotransmitter Release from Catecholaminergic Cells", THE JOURNAL OF NEUROSCIENCE, vol. 26, no. 50, 13 December 2006 (2006-12-13), US, pages 13017 - 13024, XP055849129, ISSN: 0270-6474, Retrieved from the Internet <URL:https://www.ncbi.nlm.nih.gov/pmc/articles/PMC6674961/pdf/zns13017.pdf> DOI: 10.1523/JNEUROSCI.2070-06.2006
- HASHIDA T ET AL: "Plasma membranes purified from myeloid leukemia cells before and after differentiation - I. Characterization of spectrin-like proteins and increased association of actin", EXPERIMENTAL CELL RESEARCH, ELSEVIER, AMSTERDAM, NL, vol. 164, no. 2, 1 June 1986 (1986-06-01), pages 481 - 491, XP024858051, ISSN: 0014-4827, [retrieved on 19860601], DOI: 10.1016/0014-4827(86)90046-7

## Description

### Technical field

The present invention relates to a biomarker and its use. In particular, its use in predicting the likelihood of a cancer metastasis, or in diagnosing and/or monitoring a cancer metastasis.

### Introduction

Epithelial cell cancers, known as carcinomas, account for 80 to 90 percent of all cancer cases. One such carcinoma, breast cancer, is the second most common cancer type in the world and accounts for over 10% of all cancers. Breast cancer is the most common cancer in women, with more than two million women being diagnosed annually, and over 500,000 patients dying annually of the disease.

Metastatic dissemination of a primary tumour is the leading cause of cancer related mortality, accounting for up to 90% of solid tumour cancer deaths. Additionally, patients that are diagnosed with tumours that have metastasised have a 5-year survival rate of only 22%. Metastasis occurs when cancer cells acquire a migratory epithelial-to-mesenchymal transition (EMT) phenotype, initiated from groupings of cells that disseminate from primary tumours. An invasive phenotype of such cells is a fundamental property, which correlates with their invasion to the endothelial vascular layer in the early stages of metastasis.

SUDAKOV N P ET AL: "Extracellular actin in health and disease",BIOCHEMISTRY, MAIK NAUKA - INTERPERIODICA, RU, vol. 82, no. 1, 20 January 2017 (2017-01-20), pages 1-12 discloses that membrane-bound actin was shown to be a binding-site for plasminogen. In breast cancer, overexpression of some components of the plasminogen activation cascade (urokinase) results in production of large amounts of plasmin on tumor cell surfaces.

Assessing the stage of a cancer is crucial to making any prognosis and for assigning suitable treatment regimes. Current prognosis often relies on assessing lymph node status, which involves invasive procedures such as lymph node biopsies which can lead to severe side effects such as lymphedema.

Therefore, there is a need in the art to develop new and improved methods and markers to diagnose, prognose, monitor and/or stage cancer. The present invention fulfils these needs and further provides other related advantages.

### Summary of Invention

In a first aspect, the invention provides a method of determining whether a subject who has been diagnosed with cancer is likely to develop, or is likely to have developed, a metastasis, wherein the method comprises detecting membrane-bound actin in a sample obtained from the subject, wherein it is determined that the subject is likely to develop or is likely to have developed a metastasis when membrane-bound actin is detected in the sample. The actin detected may be one or more of alpha, beta, and/or gamma actin. In an embodiment, the membrane-bound actin is beta-actin.

In a second aspect, there is provided a method of diagnosing a subject with metastatic cancer, wherein the method comprises detecting membrane-bound actin in a sample obtained from the subject, and optionally wherein it is determined that the subject has a metastatic cancer when membrane-bound actin is detected in the sample. In an embodiment, the membrane-bound actin is beta-actin.

In either of the first or second aspects, detection of any level of membrane-bound actin in the sample obtained from the subject may allow the method to be undertaken.

In a third aspect, there is provided a method of determining whether a subject who has been diagnosed with cancer is likely to develop, or is likely to have developed, a metastasis, wherein the method comprises:
a. measuring the level of membrane-bound actin in a sample obtained from the subject,
b. comparing the level of membrane-bound actin measured in the sample obtained from the subject with the level of membrane-bound actin in a reference sample, and
c. determining that the subject is likely to develop a metastasis or is likely to have developed a metastasis if the level of membrane-bound actin in the sample obtained from the subject is about equal to or higher than the level of membrane-bound actin in the reference sample.

In an embodiment, the membrane-bound actin is beta-actin.

In a fourth aspect, there is provided a method of diagnosing a subject with a metastatic cancer, wherein the method comprises:
a. measuring the level of membrane-bound actin in a sample obtained from the subject,
b. comparing the level of membrane-bound actin measured in the sample obtained from the subject with the level of membrane-bound actin in a reference sample, and
c. diagnosing the subject with a metastatic cancer when the level of membrane-bound actin in the sample obtained from the subject is about equal to or higher than the level of membrane-bound actin in the reference sample.

In an embodiment, the membrane-bound actin is beta-actin.

In a fifth aspect, there is provided a method of prognosing a subject who has been diagnosed with cancer, wherein the method comprises
a. measuring the level of membrane-bound actin in a sample obtained from the subject,
b. comparing the level of membrane-bound actin measured in the sample with the level of membrane-bound actin in a reference sample,
c. determining that the patient has a poor prognosis when the level of membrane-bound actin in the sample obtained from the subject is about equal to or higher than the level of membrane-bound actin in the reference sample.

In an embodiment, the membrane-bound actin is beta-actin.

A poor prognosis may refer to an increased likelihood of developing a metastasis and/or a reduced chance of survival of the subject.

In a sixth aspect, there is provided a method of identifying a patient who has been diagnosed with cancer who is likely to benefit from treatment with known treatments for metastatic cancer, wherein the method comprises:
a. measuring the level of membrane-bound actin in a sample obtained from the subject,
b. comparing the level of membrane-bound actin measured in the sample with the level of membrane-bound actin in a reference sample.
c. determining that the patient is likely to benefit from treatment with known treatments for metastatic cancer if the level of membrane-bound actin in the sample obtained from the subject is about equal to or higher than the level of membrane-bound actin in the reference sample.

In an embodiment, the membrane-bound actin is beta-actin.

The skilled person will be able to identify a suitable known treatment for metastatic cancer and will understand that the known treatment for metastatic cancer will vary depending on how extensive and advanced the metastatic disease is likely to be and the molecular profile of the cancer. For example, a suitable treatment may be adjuvant chemotherapy, wherein the specific treatment and/or regime depends on the characteristics of the specific tumour of the subject.

As an example, known treatments for metastatic cancer may be treatments known for use in treating metastatic breast cancer, such as Herceptin^{™} or another Her2 blocking monoclonal antibody treatment.

In any aspect, the membrane-bound actin may be detected or measured in the membrane of a cell, such as an epithelial cancer cell. The membrane bound actin may be detected or measured in the membrane of an extracellular vesicle (EV). The membrane bound actin may be detected in both the membrane of a cell and in the membrane of an extracellular vesicle.

In any aspect, an EV may include, but is not limited to, exosomes, exomeres, microvesicles, and apoptotic bodies.

The sample obtained from the subject, or used as a reference, in any aspect may be a biofluid sample such as a blood sample, saliva sample, urine sample, or the sample may be a tissue biopsy sample such as a breast tissue biopsy sample.

In any aspect, the membrane-bound actin may be detected or measured from the plasma membrane of a breast cancer epithelial cell from a solid breast tissue biopsy obtained from the subject.

In any aspect, the membrane bound actin may be detected or measured from the membrane of an extracellular vesicle isolated from a blood sample obtained from the subject.

In any of the third to sixth aspects, the reference sample may be a positive reference sample.

In any of the third to sixth aspects, the reference sample may be a negative reference sample.

In any aspect, the membrane-bound actin may be beta-actin. In any aspect, the membrane-bound actin may be alpha-actin. In any aspect, the membrane-bound actin may be gamma-actin. In any aspect, the membrane-bound actin may be one or more, or all of alpha-actin, beta-actin and gamma-actin.

When the reference sample is a positive reference sample, the determination in step c of any of the third to sixths aspects of the invention is made when the level of membrane-bound actin detected or measured in that sample is equal to or higher than the level of membrane-bound actin detected or measured in the reference sample.

When the reference sample is a negative reference sample, the determination in step c of any of the third to sixth aspects of the invention is made when the level of membrane-bound actin detected or measured in that sample is higher than the level of membrane-bound actin detected or measured in the reference sample. The magnitude of difference may depend on the relationship between the sample obtained from the subject and the reference sample, and the characteristics of each sample. For example, the determination may be made when the level of membrane-bound actin detected or measured in the sample obtained from the subject is at least about 5% more than, at least about 10% more than, at least about 20% more than, at least about 50% more than, at least about 100% more than, at least about 200% more than, at least about 300% more than, at least about 400% more than, at least about 500% more than, at least about 1000% more than, at least about 2000% more than, at least about 5000% more than, about at least 10,000% more than, at least 20,000% or more, at least 50,000% more, at least 100,000% more, at least 500,000% more, at least 1,000,000% more than the level of membrane-bound actin detected or measured in the reference sample. Alternatively or additionally the determination may be made when the level of membrane-bound actin detected or measured in the sample obtained from the subject is at least about at least about 1-fold or more, at least about 2-fold or more, at least about 3-fold or more, at least about 4-fold or more, at least about 5-fold or more, at least about 10-fold or more, at least about 20-fold or more, at least about 50-fold or more, at least about 100-fold or more, at least about 250-fold or more, at least about 500-fold or more, at least about 1000-fold or more, at least about 5000-fold or more, at least about 10,000-fold or more, at least about 20,000-fold or more, at least about 50,000-fold or more, at least about 100,000-fold or more, at least about 500,000-fold or more higher than the level of membrane-bound actin detected or measured in the reference sample.

In an embodiment of any aspect, the sample obtained from the subject may comprise epithelial cells, and/or EVs, and/or circulating tumour cells. A circulating tumour cell may be an epithelial cell.

If the sample comprises epithelial cells, the detecting of membrane-bound actin may be carried out on these cells, which may be first isolated, identified or separated from the remaining sample, if required.

If the sample comprises EVs, the detection or measurement of membrane-bound actin may be carried out on these EVs, which may be isolated, identified or separated from the remaining sample, if required.

If the sample comprises circulating tumour cells, the detection or measurement of membrane-bound actin may be carried out on these cells, which may be isolated, identified or separated from the remaining sample, if required.

If the cells and/or EVs are to be isolated, identified or separated from the remaining sample, one or more suitable biomarkers may be used.

For example, to isolate, identify or separate epithelial cells from a sample, an epithelial anti-EpCAM antibody may be used in any method the skilled person sees fit, for example FACS.

To isolate or separate EVs from a sample, one or more of anti-CD9, anti-CD63 and/or anti-CD81 antibodies may be used in any method the skilled person sees fit, for example FACS. Similarly, an anti-EpCAM antibody may be used in combination with the one or more EV-specific biomarkers to isolate, identify or separate EVs which are derived from epithelial cells from the remaining sample.

To isolate, identify or separate circulating tumour cells from a sample, an anti-EpCAM antibody or DNA or RNA which bind EpCAM may be used. This is because epithelial cells are not usually found in circulation, and their presence would signify a circulating tumour cell. To isolate, identify or separate circulating tumour cells from a sample, a means to detect one or more of ER, PR, EGFR, HER2, TOP2A may be used (Nadal et al. Breast Cancer Research 2012, 14:R71). The means may be an antibody, DNA or RNA molecule which specifically binds ER, PR, EGFR, HER2 or TOP2A.

In another aspect, there is provided a method of monitoring the response of a subject to treatment for a metastatic cancer, comprising measuring the level of membrane-bound actin in a sample obtained from the subject, wherein it is determined that the subject is responding to the treatment of the metastatic cancer when the level membrane-bound actin measured in the sample is less than the level of membrane-bound actin in a previous corresponding sample obtained from the subject. Alternatively, it may be determined that the subject is not responding to the treatment of the metastatic cancer when the level membrane-bound actin measured in the sample is equal to or more than the level of membrane-bound actin in a previous corresponding sample obtained from the subject. The previous corresponding sample may be obtained prior to or at the time treatment is administered to the subject. Preferably the levels of membrane-bound actin in a previous corresponding sample is compared to the levels in one or more samples obtained after administration of a treatment, these samples may be obtained at one or more time points after the administration of a treatment. By monitoring the levels of membrane-bound actin during the course of treatment and/or after treatment the response of the subject to the treatment can be determined. It will be appreciated that a skilled medical practitioner may use the levels of membrane-bound actin observed in samples during treatment to adjust the treatment as necessary.

In another aspect, there is provided a method of monitoring disease progression in a subject diagnosed with a metastatic cancer, comprising measuring the level of membrane-bound actin in a sample obtained from the subject, wherein it is determined that the metastatic cancer is not progressing when the level of membrane-bound actin measured in the sample is less than the level of membrane-bound actin in a previous corresponding sample obtained from the subject. Alternatively, it may be determined that the metastatic cancer is progressing when the level membrane-bound actin measured in the sample is equal to or more than the level of membrane-bound actin in a previous corresponding sample obtained from the subject.

In another aspect, there is provided a method of monitoring the reoccurrence of a metastatic cancer in a subject who had been successfully treated for the metastatic cancer, comprising measuring the level of membrane-bound actin in a sample obtained from the subject, wherein it is determined that the metastatic cancer has not reoccurred when the level of membrane-bound actin measured in the sample is less than the level of membrane-bound actin measured in a previous corresponding sample obtained from the subject. Alternatively, it may be determined that the metastatic cancer has reoccurred when the level membrane-bound actin measured in the sample is equal to or more than the level of membrane-bound actin measured in a previous corresponding sample obtained from the subject. The corresponding sample may have been taken at the point of, or within one week, two weeks or four weeks of determination of the successful treatment.

Reference herein to a "corresponding sample" may refer to a sample of the same type, for example same tissue type, taken from the same subject. The sample is preferably taken at an earlier time point.

The method of any aspect of the invention may be performed *in vivo, ex vivo* or *in vitro.* Preferably, the method is an *in vitro* method.

In a further aspect, there is provided a kit (not claimed) for (a) determining whether a subject who has been diagnosed with cancer is likely to develop or is likely to have developed a metastasis, (b) prognosing a subject diagnosed with cancer, (c) diagnosing a subject with metastatic cancer, (d) monitoring the response of a subject to the treatment for a metastatic cancer, or (e) monitoring disease progression in a subject diagnosed with a metastatic cancer,
wherein the kit comprises a means for detecting membrane-bound actin, and optionally further comprises one or more of:
i. means for identifying a cell type of interest, and/or
ii. reagents for isolating EVs.

In an embodiment, the membrane-bound actin is beta-actin.

The means for detecting membrane-bound actin may be an actin binding polypeptide, such as an antibody. For example, the means for detecting membrane-bound actin may be an anti actin antibody, such as an anti beta-actin antibody. The means for detecting membrane-bound actin may be a DNA or RNA molecule which is capable of binding actin, such as an aptamer.

The cell type of interest may be an epithelial cell or a circulating tumour cell. The circulating tumour cell may be an epithelial tumour cell.

The means for identifying the cell type of interest may be an EpCAM binding polypeptide, such as an antibody. The means for identifying the cell type of interest may be an RNA or DNA molecule which is capable of binding EpCAM, such as an aptamer.

In an embodiment, the kit may further comprise reagents for isolating EVs from a sample. The skilled person will be able to choose any set of reagents which are available in the art to isolate EVs from a sample. The exact reagents chosen may depend on the nature of the sample, for example specific reagents may be chosen which are particularly compatible with blood samples.

In an embodiment, the kit may further comprise a set of instructions.

In a certain non-limiting embodiment, the means for detecting membrane-bound actin and/or the means for identifying the cell type of interest may be provided bound to a solid support, such as a column matrix, an array, or well of a microtiter plate. Alternatively, the support can be provided as a separate element of the kit.

As used herein, the terms "actin binding polypeptide" and "EpCAM binding polypeptide" may refer to any polypeptide which binds actin or EpCAM, respectively. Such a polypeptide may be a T-cell receptor, or antibody or antigen binding fragment thereof, including, but not limited to Fv, Fab, Fab' and F(ab')2 fragments, and single domain antibodies such as camelid single domain antibodies. Antibodies or antigen binding fragments, both polyclonal and monoclonal, specific for actin such as beta-actin, and/or specific for EpCAM, can be prepared using conventional immunization techniques, as will be generally known to those of skill in the art.

The means for identifying the cell type of interest may also comprise DNA or RNA molecules capable of binding EpCAM, such as aptamers.

Antibodies, polypeptides, or DNA or RNA molecules referred to herein may comprise detectable labels that are associated with, or linked to the given antibody, polypeptide or DNA or RNA molecule. Such detectable labels include, for example, chemiluminescent or fluorescent molecules (for example rhodamine, fluorescein, green fluorescent protein, luciferase, Cy3, Cy5 or ROX), radiolabels (for example 3H, 35S, 32P, 14C, 131I), enzymes (for example alkaline phosphatase, horseradish peroxidase), or peptide/amino acid sequences (for example His-Tag, FLAG, Myc).

In any aspect or embodiment, the cancer may be or may be derived from an epithelial cell cancer, such as breast cancer, colorectal cancer, lung cancer or pancreatic cancer.

The metastatic cancer may be metastatic breast cancer, metastatic colorectal cancer, metastatic lung cancer or metastatic pancreatic cancer.

HPA or Helix pomatia agglutinin is a lectin derived from Helix pomatia which can be used to detect glycans that have a terminal N-acetylgalactosamine (GalNAc). As used herein, "HPA positive" and "HPA positivity" refers to a cell or EV that displays significant levels of GalNAc on its surface and is therefore recognised by, and binds high levels of HPA. An HPA negative cell or EV is defined as a cell that does not display or displays low levels of GalNAc on its surface, and is therefore not recognised by, and does not bind to, HPA or binds only low levels of HPA.

A sample comprising cells and/or EVs in a sample may be identified as HPA positive if more than about 50%, 60%, 70%, 80%, 90%, 95%, 98%, 99% of cells and/or EVs in the sample are HPA positive.

A sample comprising cells and/or EVs in a sample may be identified as HPA negative if more than about 50%, 60%, 70%, 80%, 90%, 95%, 98%, 99% of cells and/or EVs in the sample are HPA negative.

In any aspect, the invention may further comprise detecting or measuring the level of HPA positivity of cells and/or EVs.

In any aspect, the invention may further or alternatively comprise detecting or measuring the level of HPA positivity in addition to or as an alternative to detecting or measuring actin. HPA positivity may be determined on EVs. For example, in a method of determining whether a subject who has been diagnosed with cancer is likely to develop, or is likely to have developed, a metastasis, the method may comprise:
a. measuring the level of membrane-bound actin and/or HPA positivity in a sample obtained from the subject,
b. comparing the level of membrane-bound actin and/or HPA positivity measured in the sample obtained from the subject with the level of membrane-bound actin and/or HPA positivity in a reference sample, and
c. determining that the subject is likely to develop a metastasis or is likely to have developed a metastasis if the level of membrane-bound actin and/or HPA positivity in the sample obtained from the subject is about equal to or higher than the level of membrane-bound actin and/or HPA positivity in the reference sample. In such a method, EVs may be isolated, identified or separated from the remaining sample, if required.

### DETAILED DESCRIPTION

The invention is in part based on the finding of a positive correlation between the presence and/or level of membrane-bound actin on the surface of epithelial cancer cells and/or extracellular vesicles derived therefrom, and the metastatic potential of these cancer cells.

Therefore determining the presence of, or increased level of actin on the cell surface, or in the membrane of an extracellular vesicle, could permit clinicians to accurately identify the metastatic potential of cancer cells and to stage and/or prognose a cancer. This would allow identification of later-stage breast cancer patients or those likely to develop or have already developed a metastasis so they can receive faster and more appropriate treatment. The method could also be used to monitor patient response to treatment and/or to monitor disease progression.

Additionally, the invention may allow patients to avoid unnecessary lymph node biopsies. For example, the invention may be performed on cells from tissue derived from or surrounding the primary tumour, such as breast tissue from an initial biopsy which is used to make a diagnosis of breast cancer, or after removal of breast tissue during therapy. Additionally or alternatively, when detecting or determining the level of actin in the membrane of an extracellular vesicle, the invention may be performed on easily accessible biofluids without requiring a biopsy. The invention may allow the determination of metastatic potential, staging of the cancer, diagnosis, prognosis and/or identification of a patient requiring a specific treatment to be performed quickly, less or non-invasively, and/or prior to surgical intervention.

The skilled person will understand that a number of methods or techniques in the art can be used to detect or measure the level of membrane-bound actin. Suitable techniques include immunohistochemistry, immunocytochemistry, flow cytometry, ELISA, lateral flow assay, western blot following biochemical or mechanical separation/isolation of membranes, dot-blots, slot-blots, and mass spectrometry. **In** any aspect, the detection of actin may be direct, or indirect, for example using a primary detection reagent which detects actin, and a secondary detection reagent which specifically detects the primary detection reagent. The secondary detection reagent may be labelled. The invention may therefore be widely adopted at many hospitals or laboratories globally, without the need for highly specialised equipment or training.

In an embodiment the level of actin may be detected by forming a complex of actin with a detection agent and then detecting the actin/detection agent complex.

The skilled person will understand that a number of methods or techniques in the art can be used to isolate EVs from a sample (reviewed in Konoshenko et al., 2018, BioMed. Res. Intl.).

Actin as referred to herein is the protein which is endogenous to the host species. In humans, for example, actin may refer to beta-actin which is the protein encoded by the ACTB gene. Actin may refer to alpha-actin which is the protein encoded by the ACTA1 or ACTA2 genes. Actin may refer to gamma-actin which is the protein encoded by the ACTG1 or ACTG2 genes.

"Membrane bound actin" as used herein refers to actin, such as beta-actin which is present or detectable on the surface of a cell, preferably in/on the plasma membrane of the cell, or which is present or detectable on the surface of an extracellular vesicle. The actin may have post-translational modifications such as glycosylation marks. The actin may be detectable without having to disrupt, chemically or mechanically, the cell and/or EV. The actin may be detectable in membranous fractions of cells and/or EVs that have been mechanically and/or chemically disrupted, wherein said fractions comprise membrane only of the plasma membrane of cells or of the EV. The actin which is detected or measured may be an integral membrane protein or a peripheral membrane protein which may interact with an integral membrane protein.

"Likely to develop, or is likely to have developed, a metastasis" as used herein refers to an increased likelihood of developing or having already developed a metastasis as compared with a subject who does not have the required characteristics set forth in the aspects and/or embodiments of the invention.

As used herein, the term "higher" may refer to a number which is at least about 5% or more, at least about 10% or more, at least about 20% or more, at least about 50% or more, at least about 100% or more, at least about 200% or more, at least about 300% or more, at least about 400% or more, at least about 500% or more, at least about 1000% or more, at least about 2000% or more, at least about 5000% or more, at least about 10000% or more, at least about 20000% or more, at least about 50000% or more, at least about 100000% or more, at least about 500000% or more, at least about 1000000% or more higher than the level of membrane-bound actin in the reference sample. Alternatively, the term "higher" may refer to a number which is at least about 1-fold or more, at least about 2-fold or more, at least about 3-fold or more, at least about 4-fold or more, at least about 5-fold or more, at least about 10-fold or more, at least about 20-fold or more, at least about 50-fold or more, at least about 100-fold or more, at least about 250-fold or more, at least about 500-fold or more, at least about 1000-fold or more, at least about 5000-fold or more, at least about 10000-fold or more, at least about 20,000-fold or more, at least about 50,000-fold or more, at least about 100000-fold or more, at least about 500000-fold or more higher than the level of membrane-bound actin in the reference sample.

As used herein, the terms "actin binding polypeptide" and "EpCAM binding polypeptide" may refer to any polypeptide which binds actin or EpCAM, respectively. Such a polypeptide may be a T-cell receptor, or antibody or antigen binding fragment thereof, including, but not limited to Fv, Fab, Fab' and F(ab')2 fragments.

As used herein, the term "sample obtained from a subject" refers to a sample of biological material obtained from a subject, e.g., a human subject, including tissue, a tissue sample, a biopsy, a cell sample, a tumour sample, a stool sample and a sample of biological fluid, e.g., plasma, serum, blood, urine, lymphatic fluid, ascites, saliva. In an embodiment, the sample is a tissue sample, for example from a biopsy of breast tissue from a subject. In an embodiment, the sample is a blood sample obtained from a subject.

The term "patient" or "subject," as used interchangeably herein, refers to any mammal, e.g., a human. Non-limiting examples of non-human mammals include non-human primates, dogs, cats, mice, rats, guinea pigs, rabbits, fowl, pigs, horses, cows, goats, sheep, etc.

In the present disclosure, a sample which is described as a "biopsy" can be obtained by conventional methods, using methods well known by the persons skilled in related medical techniques. The methods for obtaining a biopsy sample include splitting a tumour into large pieces, or microdissection, or other cell separating methods known in the art. The tumour cells can additionally be obtained by means of cytology through aspiration with a small gauge needle. To simplify sample preservation and handling, samples can be fixed in formalin and soaked in paraffin or first frozen and then soaked in a tissue freezing medium such as OCT compound by means of immersion in a highly cryogenic medium which allows rapid freezing. Other methods to preserve and/or process samples are known to those skilled in the art.

The sample can be manipulated to isolate cell types of interest or to isolate EVs. For example, a marker for a cell type of interest in a tissue sample or biopsy can be used to isolate or separate such cells. An example is the use of an EpCAM binding polypeptide to identify and/or separate epithelial cells from tissue taken in a biopsy, before membrane-bound actin, such as beta-actin, is detected or the level determined. Another example is the use of an EpCAM binding polypeptide to identify and/or separate EVs derived from epithelial cells from other EVs and/or biological material in the sample.

A reference sample may refer to an equivalent sample, for example the same tissue and/or cell type as the sample obtained from the subject or from other subjects, or from a cell line which closely resembles the sample type obtained from the subject.

A "negative reference sample" may refer to a reference sample which is non-cancerous, or which is cancerous/immortalised but which is known to have not developed or to not develop metastasis. For example, a negative reference sample may be a corresponding sample, such as a biopsy sample, from the area surrounding a tumour of the subject, or corresponding tissue from one or more healthy subjects. A negative reference sample may also be a cell line which is known to be non-metastatic in nature and which is of the same or similar nature to the sample obtained for the subject, for example a breast epithelial cell line which is known to be low in invasiveness such as BT474 cells (Lasfargues et al., 1978, J. Nat. Cancer Institute, 61(4): 967-978) as a negative reference sample for a breast tissue sample obtained from the subject, or HMT3522 Human breast epithelial cells isolated from benign fibrocystic breast tissue (Briand et al, 1987, In Vitro Cell and Dev. Biol., 23 (3): 181-188).

Where the presence or level of membrane-bound actin is to be detected or measured in EVs, a negative reference sample may refer to EVs isolated from one or more subjects who do not have cancer, or who have/had cancer and have not or did not develop metastasis, or from a comparative cell line which is known to be low in invasiveness, as above.

Thus, the level of membrane-bound actin detected or measured in such a negative reference sample acts as a negative control.

A "positive reference sample" may refer to a reference sample which is cancerous, which is known to be invasive, and/or which is known to have developed or to be likely to develop metastasis. For example, a positive reference sample may be a corresponding sample, such as a biopsy sample, from one or more subjects known to have developed metastasis. A positive reference sample may also be a cell line which is known to be invasive and/or metastatic in nature and which is of the same or similar nature to the sample obtained for the subject, for example a breast epithelial cell line which is known to be high in invasiveness such as MCF7 cells (Soule et al., 1973, J. Nat. Cancer Institute, 51(5): 1409-14-16) as a positive reference sample for a breast tissue sample obtained from the subject.

Where the presence or level of membrane-bound actin is to be detected or measured in EVs, a positive reference sample may refer to EVs isolated from one or more subjects who have/had cancer, and who developed metastasis, or from a comparative cell line which is known to be high in invasiveness, as above.

Thus, the level of membrane-bound actin detectable or measured in such a positive reference sample acts as a positive control.

A sample from a subject who has been diagnosed with cancer and in which higher levels of membrane-bound beta-actin are measured than in those of the above reference samples may be identified as being more likely to develop or to have developed metastasis or to be diagnosed with metastatic cancer. A suitable treatment regime can then be identified and provided to the patient.

The skilled person could consult a database in which the level of membrane-bound actin in a given sample type has been recorded from subjects with confirmed metastasis or no recorded metastasis, or from cell lines which are known to be metastatic or non-metastatic in nature. The skilled person is easily able to identify such metastatic or non-metastatic cell lines, where appropriate.

The skilled person will appreciate that preferred features of any one embodiment and/or aspect of the invention may be applied to all other embodiments and/or aspects of the invention.

### BRIEF DESCRIPTION OF THE FIGURES

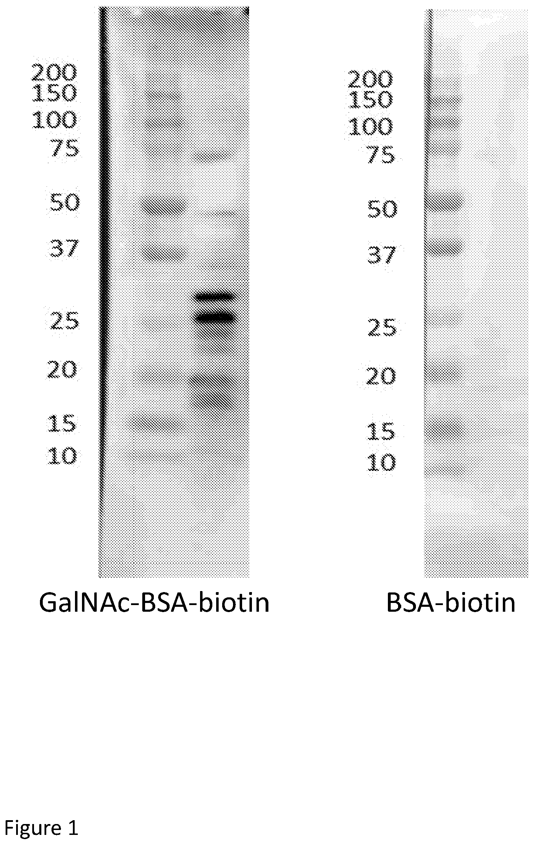

**Figure 1** **- shows that GalNAc binds to multiple proteins in endothelial cells using 1D western blot. A.** Total endothelial cell protein was extracted and run on SDS-PAGE then western blot performed. Membrane on the left was probed with GalNAc-BSA-biotin and revealed several bands. As a negative control the membrane on the right was labelled with BSA-biotin and no bands were observed.

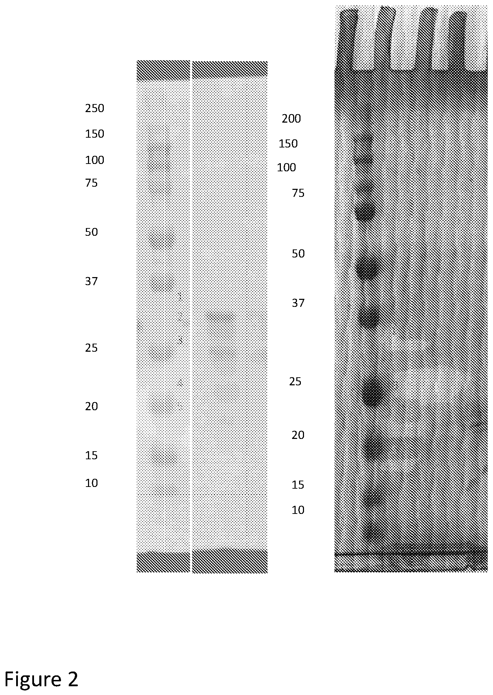

**Figure 2** **- shows bands excised for analysis by mass spectrometry.** 1D SDS-PAGE and blot indicating where gel fragments were excised for mass spec analysis, 5 fragments were analysed.

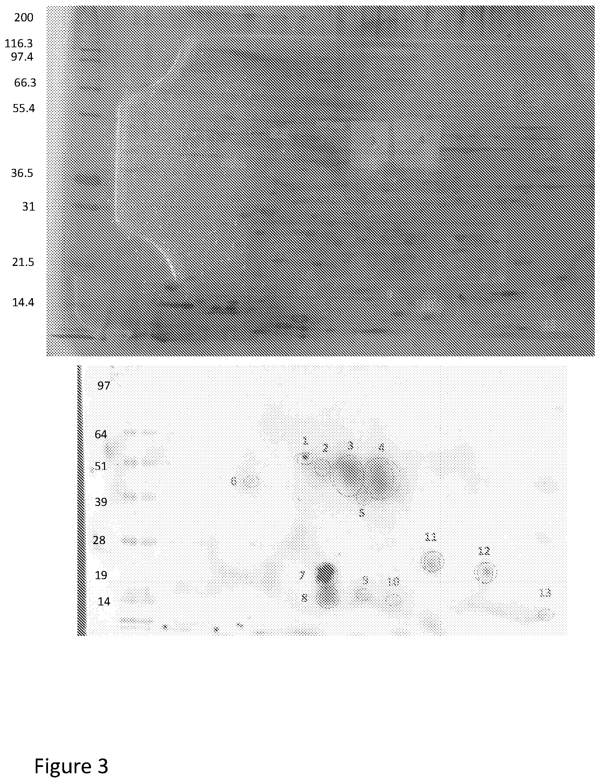

**Figure 3** **- shows that GalNAc binds to multiple proteins in endothelial cells using 2D PAGE.** 2D SDS-PAGE and blot indicating where gel fragments were excised for mass spec, 13 fragments in total.

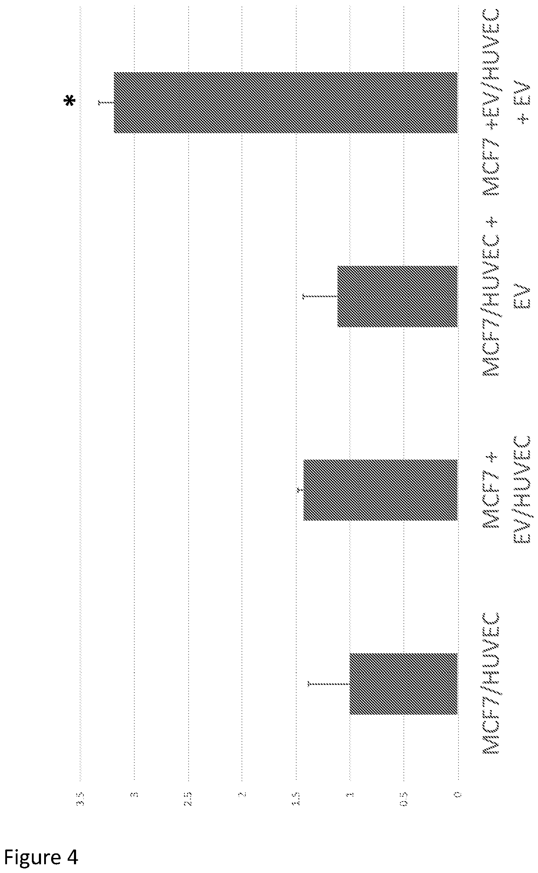

**Figure 4** **- shows that EVs can increase cellular adhesion.** EVs were isolated from breast cancer cells and incubated with either breast cancer cells, endothelial cells, or both, prior to the static adhesion assay. Breast cancer cell adhesion to endothelial cells is significantly increased in the presence of EVs. The largest increase is seen when both breast cancer cells and endothelial cells are pre-treated with EVs. Data from three replicates, error bars are standard error of the mean (SEM) and statistical test performed is one-way ANOVA with Tukey test. Star indicates significance compared to untreated control.

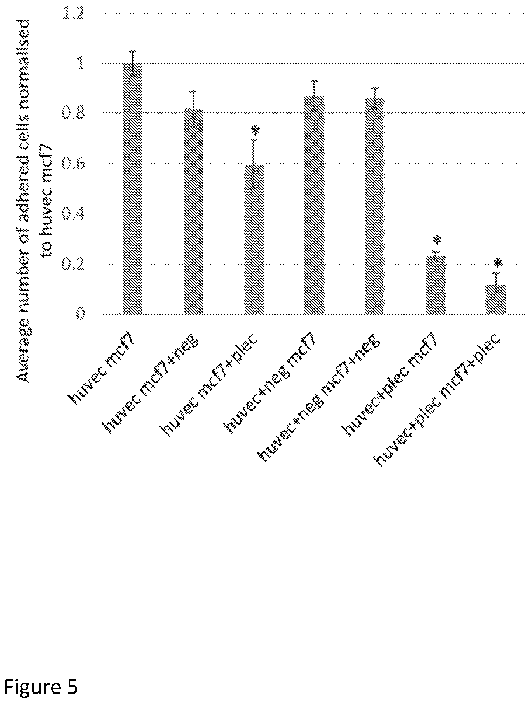

**Figure 5** **- shows that knockdown of plectin reduces adhesion of breast cancer cells to endothelial cells.** MCF7 and/or HUVEC cells were treated with negative scrambles siRNA or siRNA targeting plectin. Cell adhesion is significantly decreased when plectin is knocked down in either cell line or both cell lines. Data from three replicates, error bars are standard error of the mean and statistical test performed is one-way ANOVA with Tukey test. Star indicates statistical significance compared to untreated control.

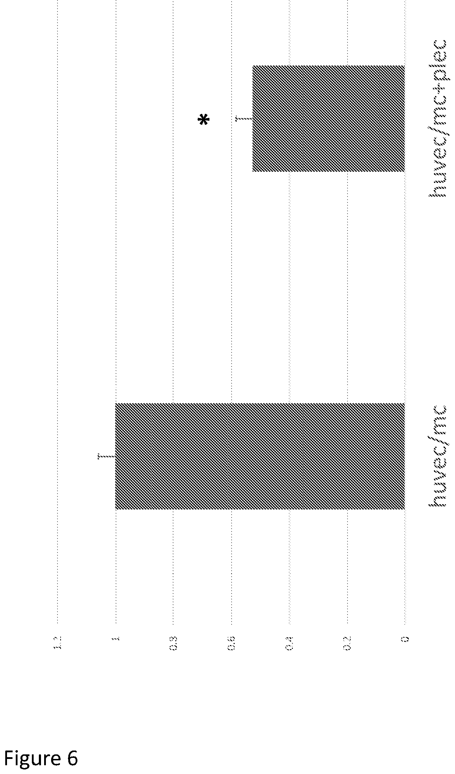

**Figure 6** **- shows that treatment of breast cancer cells with recombinant plectin reduces cell adhesion to endothelial cells.** MCF7 cells were untreated or treated with 0.1ug/ml recombinant plectin before performing a static adhesion assay. Treatment of MCF7 cells with recombinant plectin significantly decreases cell adhesion to endothelial cells. Data from three replicates, error bars are standard error of the mean and statistical test performed is one-way ANOVA with Tukey test.

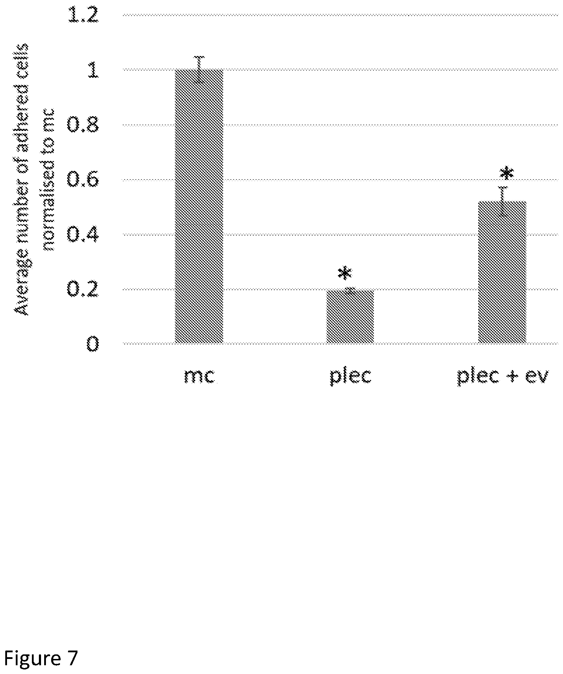

**Figure 7** **- demonstrates that in cancer cells with plectin knocked down, cell adhesion can be partially recovered with EVs from 'wild-type' cancer cells.** Plectin was knocked down using siRNA in MCF7 cells (second column) displays reduced adhesion to endothelial cells. Plectin-knockdown MCF7 cells treated with EVs from wild-type MCF7 cells show a partial recovery in adhesion to endothelial cells. Data from three replicates, error bars are standard error of the mean and statistical test performed is one-way ANOVA with Tukey test.

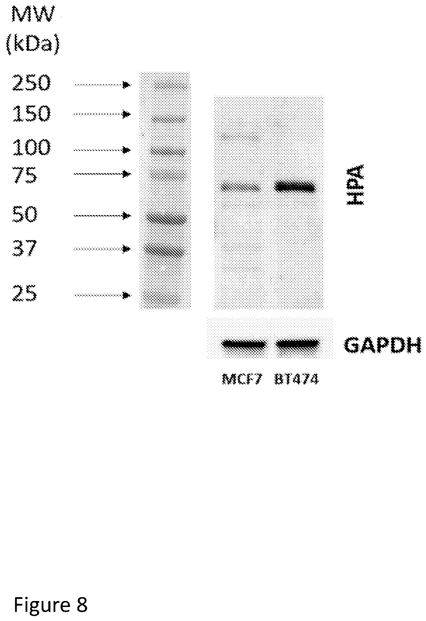

**Figure 8** **- shows that MCF7 cells have more HPA-binding proteins than BT474 cells.** Lysate from the two breast cancer cell lines was run on SDS-PAGE before western blotting using biotinylated-HPA as a probe and anti GAPDH antibody as used as a loading control. MCF7 lysate contained a greater number of HPA-binding bands than lysate from BT474 cells, which only had one HPA-binding band at 72kDa.

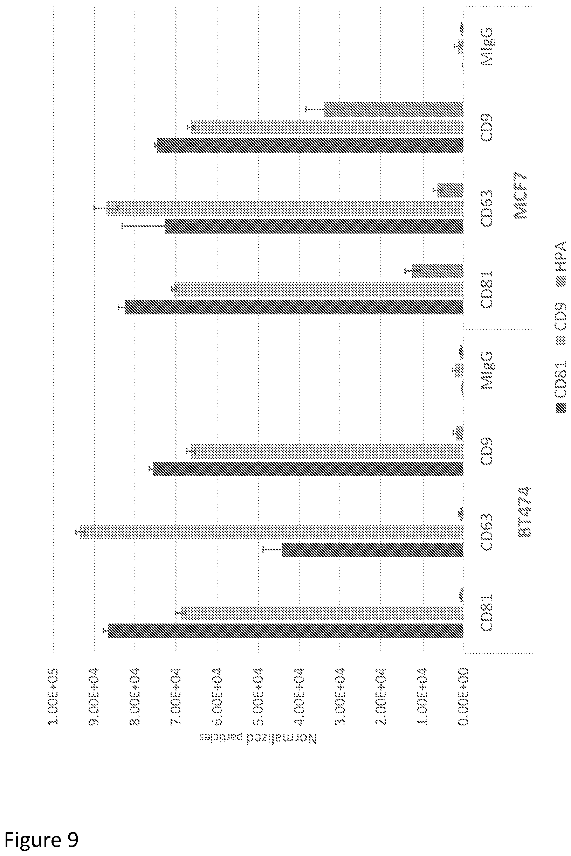

**Figure 9** **- shows a positive correlation between HPA binding capacity in cells and HPA binding capacity of EVs produced by those cells.** Nanoview analysis of MCF7 and BT474 cells and EVs produced by these cells. HPA negative BC cell line (BT474, left) and HPA-positive BC cell line (MCF7, right) EVs were labelled with fluorescently conjugated antibodies (first column = CD81, second column = CD9, third column = HPA). BT474 EVs were found to be HPA negative while MCF7 EVs were HPA positive. MigG refers to a negative control to show levels of unspecific binding.

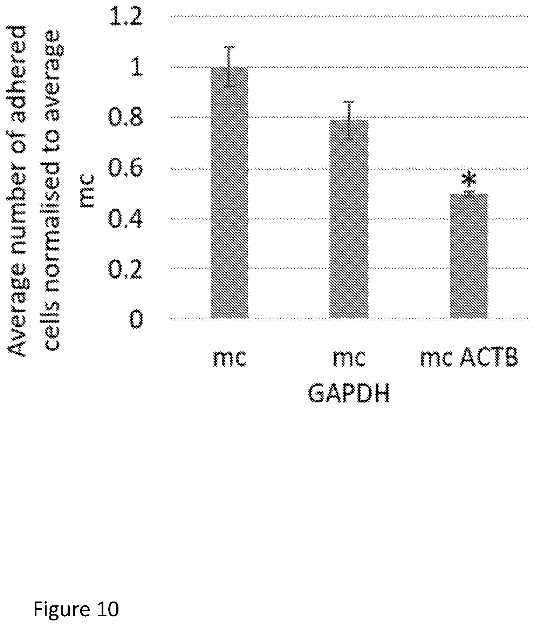

**Figure 10** **- demonstrates that blocking cell surface beta-actin reduced breast cancer cell adhesion to endothelial cells.** MCF7 cells were untreated or treated with an antibody against beta-actin or GAPDH. GAPDH is used as a control. Compared to non-treated MCF7 BC cells, cells incubated with anti-beta actin antibodies adhere significantly less to endothelial cells. Data from three replicates, error bars are SEM.

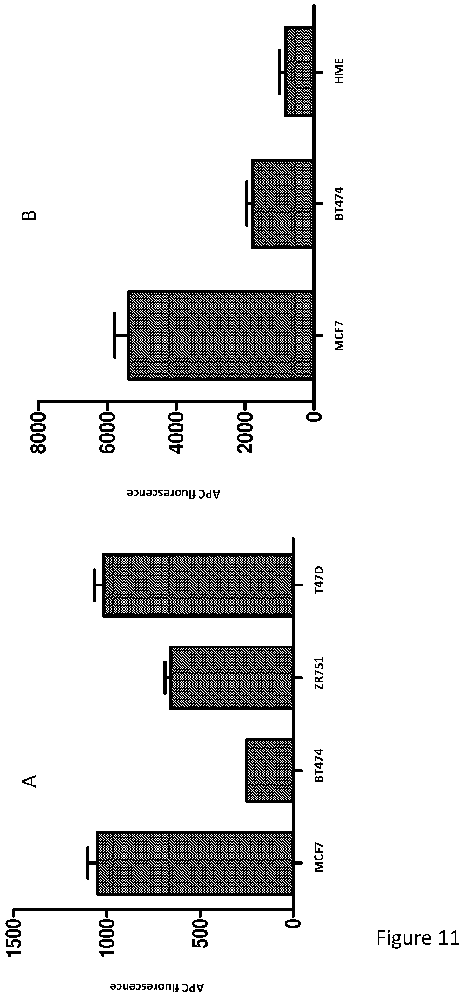

**Figure 11** **- shows that cell surface beta-actin levels correlate with HPA-binding capacity/metastatic potential.** Flow cytometry was used to detect surface actin. (A): show expression of beta-actin on the surface of highly metastatic and HPA-positive MCF7 BC cells is greater than on against non-metastatic and weakly HPA-positive BT474 cells, as well as moderately invasive and moderately HPA-positive ZR751 BC cells. Expression of beta-actin on the surface of highly metastatic and HPA-positive MCF7 BC cells is comparable to invasive and highly HPA-positive T47D BC cells. (B) shows expression of surface beta-actin on MCF7 and BT474 cells is much higher than on HPA-negative, normal breast cells HME.

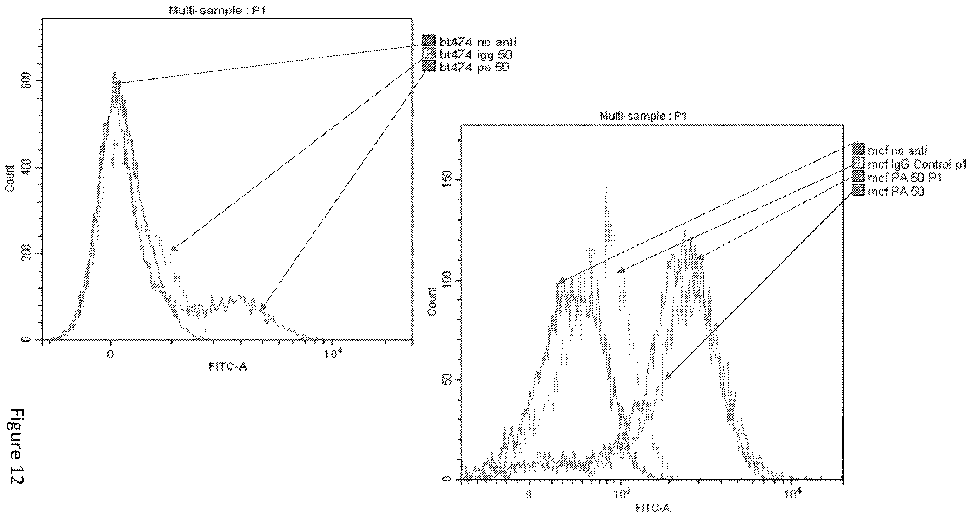

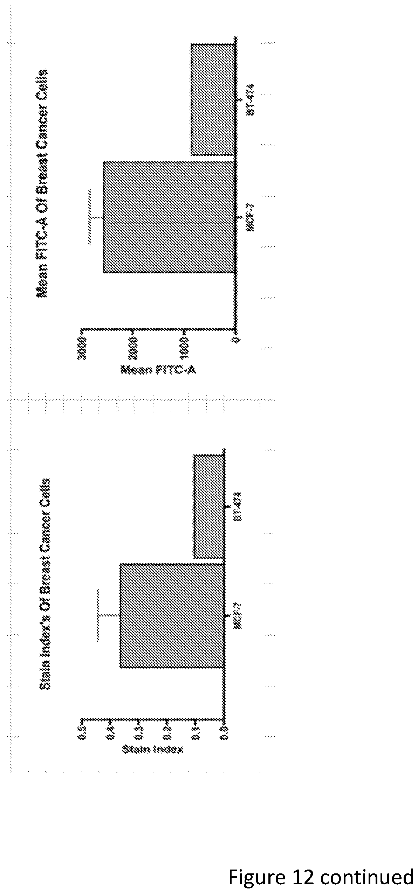

**Figure 12** **- shows that metastatic breast cancer cells (MCF7) have more actin on their surface than the non-metastatic breast cancer cells (BT474).** The data was collected by flow cytometry. Pa refers to the actin antibody that targets pan-actin. IgG refers to a negative control to show levels of unspecific binding. No anti refers to no antibody being added to show background autofluorescence.

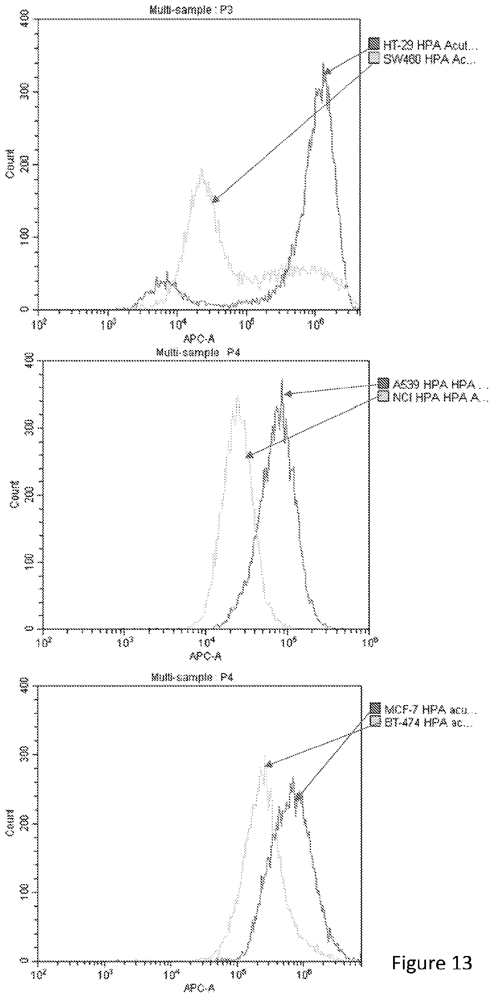

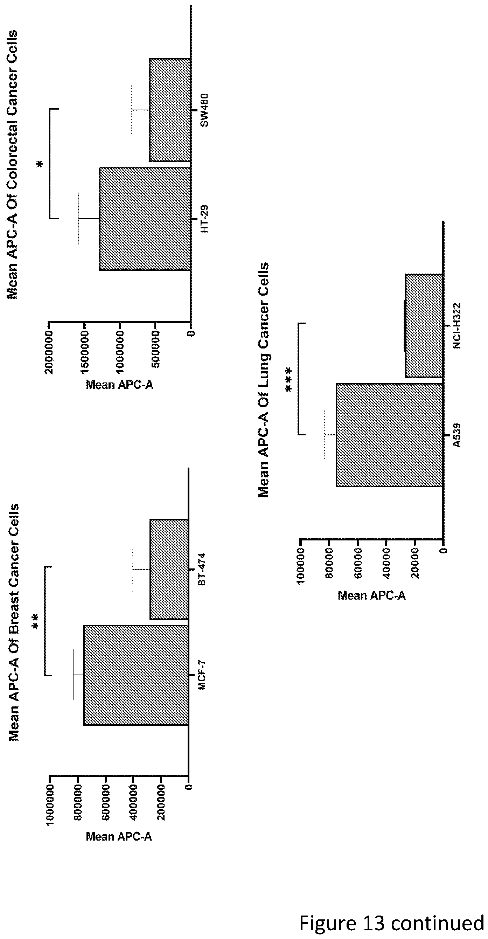

**Figure 13** **- shows that metastatic cancer cells have more HPA on their surface than the non-metastatic cancer cells.** The data was collected by flow cytometry for pairs of metastatic and non-metastatic cell types for breast (MCF7 is metastatic and BT-474 is non-metastatic), colorectal (SW480 is metastatic and HT-29 is non-metastatic) and lung cancer (A539 is metastatic and NCI-H322 is non-metastatic).

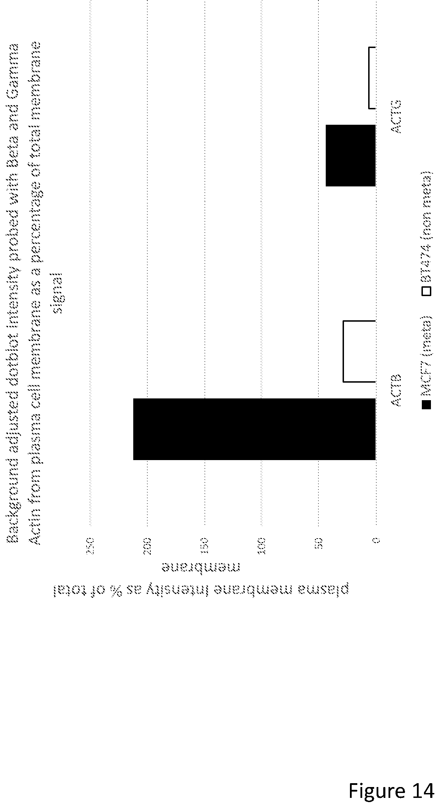

**Figure 14** **- shows that metastatic cell plasma membranes have more actin than non-metastatic cells.** The data was obtained by dotblot from probed membrane extractions of the cell lines. ACTB is an antibody that recognises actin beta, and ACTG is an antibody that recognises actin gamma. Results are depicted as the intensity of the signal adjusted to background, and normalised to the signal from total membrane.

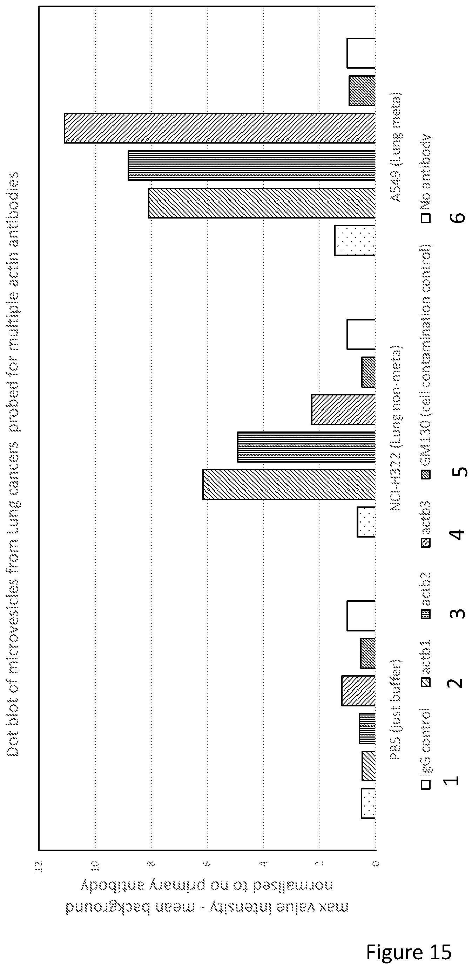

**Figure 15** **- shows that lung metastatic cancer microvesicles have more actin than those from non-metastatic cells.** The data was obtained by dotblot. The data shows the level of binding of various different antibodies to microvesicles from either metastatic lung cancer (A549) or non-metastatic lung cancer (NCI-H322). For each cancer type bars 1, 5 and 6 are controls, IgG, Gm130 and no antibody respectively, and bars 2, 3, and 4 represent three different clones of beta actin antibodies. The results presented clearly show there to be more membrane bound actin on microvesicles obtained from metastatic lung cancer cells. The results are depicted as the intensity of the signal adjusted to background and normalised to no primary antibody.

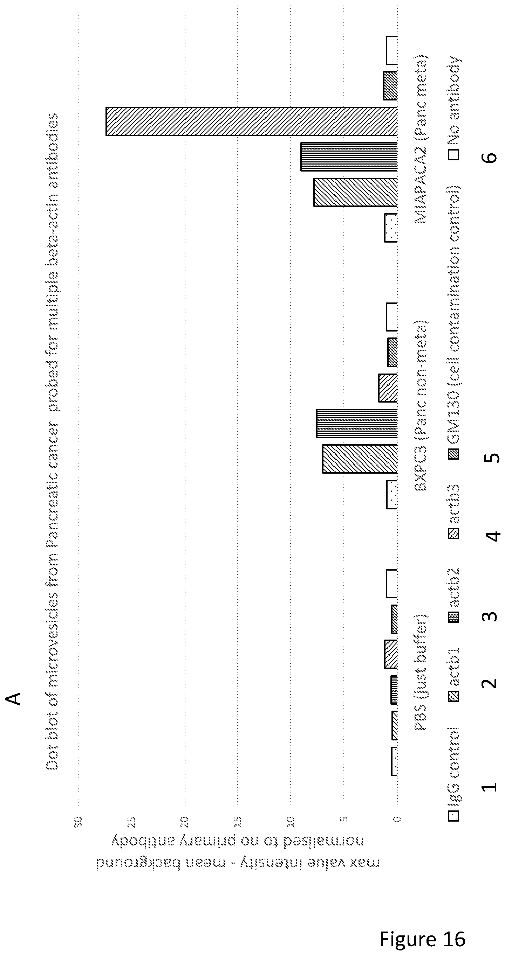

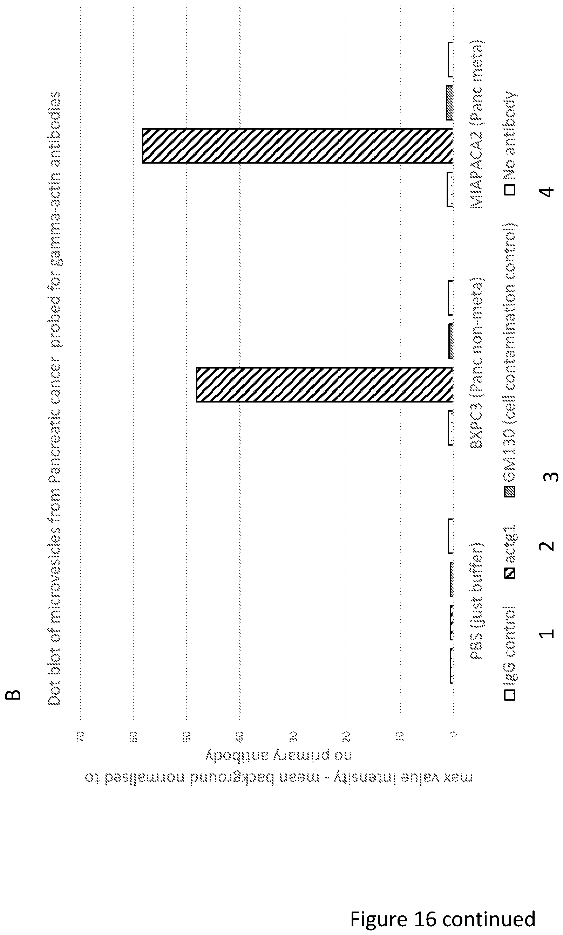

**Figure 16** **- shows that pancreatic metastatic cancer microvesicles have more actin than non-metastatic cells.** The data was obtained by dotblot. **Figure 16A** - The data shows the level of binding of various different antibodies to microvesicles from either metastatic pancreatic cancer (MIAPaCa2) or non-metastatic pancreatic cancer (BXPC-3). For each cancer type bars 1, 5 and 6 are controls - IgG, Gm130 and no antibody respectively, and bars 2, 3, and 4 represent three different beta actin antibodies. The results presented clearly show there to be more membrane bound actin on microvesicles obtained from metastatic pancreatic cancer cells.
**Figure 16B** - The data shows the level of binding of various different antibodies to microvesicles from either metastatic pancreatic cancer (MIAPaCa2) or non-metastatic pancreatic cancer (BXPC-3). For each cancer type bars 1, 3 and 4 are controls - IgG, Gm130 and no antibody respectively, and bar 2 is a gamma actin antibody. The results presented clearly show there to be more membrane bound gamma actin on microvesicles obtained from metastatic pancreatic cancer cells. Results are depicted as the intensity of the signal adjusted to background and normalised to no primary antibody.

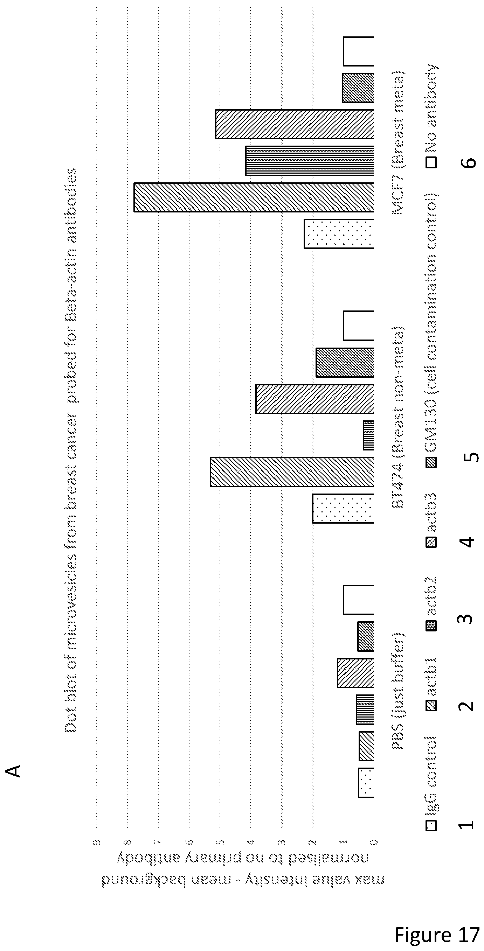

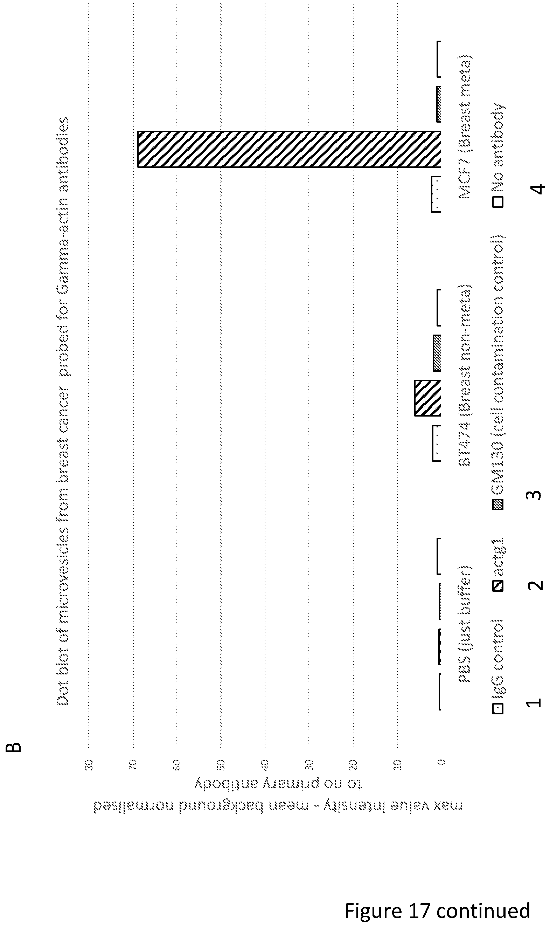

**Figure 17** **- shows that breast metastatic cancer microvesicles have more actin than non-metastatic cells.** The data was obtained by dotblot. **Figure 17A** - The data shows the level of binding of various different antibodies to microvesicles from either metastatic breast cancer (MCF7) or non-metastatic breast cancer (BT474). For each cancer type bars 1, 5 and 6 are controls - IgG, Gm130 and no antibody respectively, and bars 2, 3, and 4 represent three different beta actin antibodies. The results presented clearly show there to be more membrane bound actin on microvesicles obtained from metastatic lung cancer cells.
**Figure 17B** - The data shows the level of binding of various different antibodies to microvesicles from either a metastatic breast cancer or a non-metastatic breast cancer. For each cancer type bars 1, 3 and 4 are controls - IgG, Gm130 and no antibody respectively, and bar 2 is a gamma actin antibody. The results presented clearly show there to be more membrane bound. Results are depicted as the intensity of the signal adjusted to background and normalised to no primary antibody.

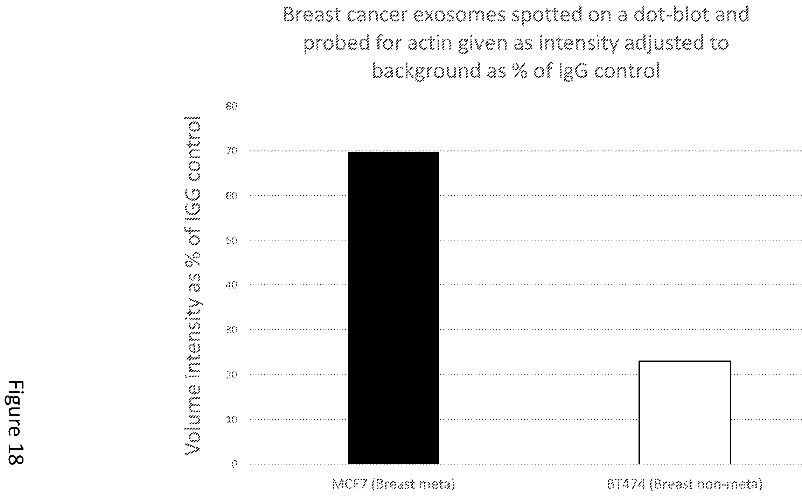

**Figure 18** **- shows that exosomes derived from metastatic breast cancer cells have more actin than exosomes derived from non-metastatic breast cancer cells.** The data was obtained by dot blot. Results are depicted as the intensity of the signal normalised to IgG control.

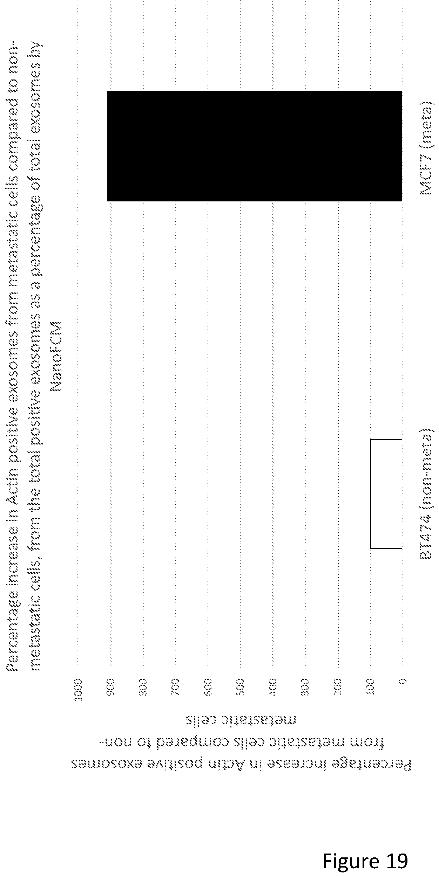

**Figure 19** **- shows that a much larger percentage of exosomes derived from metastatic breast cancer cells are actin positive than exosomes derived from non-metastatic breast cancer cells.** The data was obtained by NanoFCM. This is a single particle detection method which permits a direct percentage of population comparison, supporting the dot-blot average of total signal. Samples were probed with a pan-actin antibody.

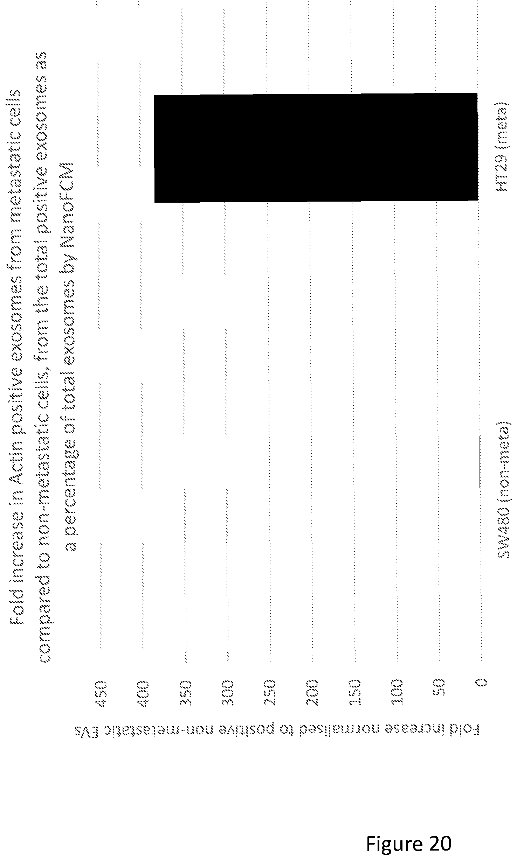

**Figure 20** **- shows that a larger percentage of exosomes derived from metastatic colorectal cancer cells are actin positive than exosomes derived from non-metastatic colorectal cancer cells.** The data was obtained by NanoFCM. This is a single particle detection method which permits a direct percentage of population comparison, supporting the dot-blots average of total signal. Samples were probed with a pan-actin antibody. Metastatic percentage of positive EVs is represented as fold change over non-metastatic percentage.

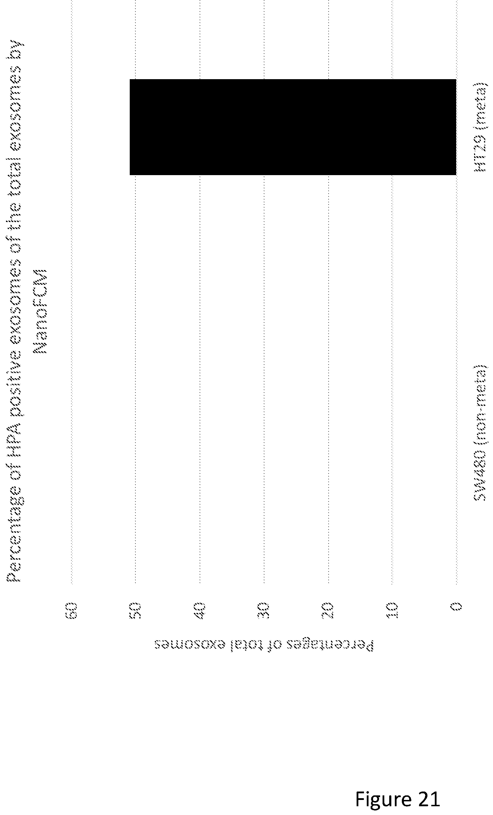

**Figure 21** **- shows that a larger percentage of exosomes derived from metastatic colorectal cancer cells are HPA positive than exosomes derived from non-metastatic colorectal cancer cells.** The data was obtained by NanoFCM, as an independent single particle analysis method to that of Nanoview, supporting the findings of Figure 9 in a different tissue type.

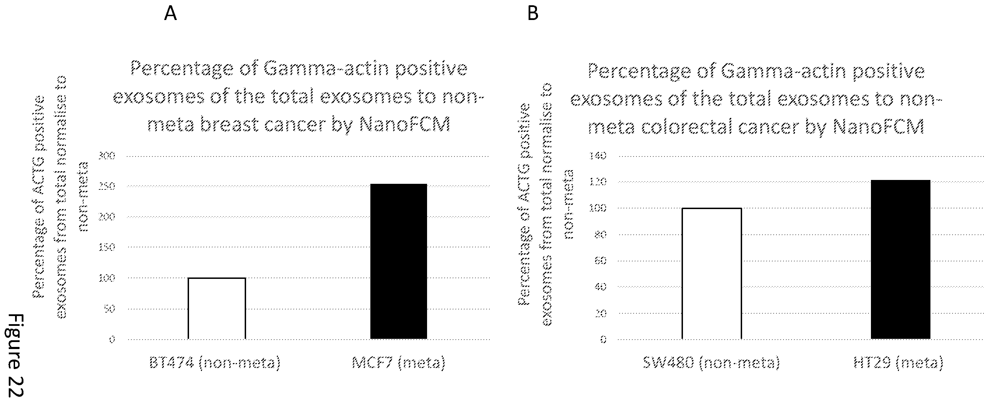

**Figure 22** **- shows that a larger percentage of (A) exosomes derived from metastatic breast cells and (B) exosomes derived from metastatic colorectal cancer cells are gamma actin positive than exosomes derived from equivalent non-metastatic cancer cells.** The data was obtained by NanoFCM.

### MATERIALS AND METHODS

**Table 1 - Cell culture**

| Cells were maintained at 37°C with 5% CO₂ atmosphere under the following conditions. | | |
|---|---|---|
| Cell line | Basal medium | Supplements |
| MCF7 | DMEM:F-12 (Lonza) | • Heat inactivated foetal calf serum (FCS) 10% v/v (Gibco^{™}) |
| | | • L-Glutamine 2mM (Gibco^{™}) |
| T47D | DMEM:F-12 (Lonza) | • Heat inactivated foetal calf serum (FCS) 10% v/v (Gibco^{™}) |
| | | • L-Glutamine 2mM (Gibco^{™}) |
| ZR751 | RPMI-1640 (Gibco^{™}) | • Heat inactivated FCS 10% v/v (Gibco^{™}) |
| | | • L-Glutamine 2mM (Gibco^{™}) |
| | | • Sodium pyruvate 1mM (Sigma-Aldrich^{®}) |
| BT474 | RPMI-1640 (Gibco^{™}) | • Heat inactivated FCS 10% v/v (Gibco^{™}) |
| | | • L-Glutamine 2mM (Gibco^{™}) |
| | | • Insulin 0.1% v/v (Sigma-Aldrich^{®}) |
| HME | DMEM:F-12 (Lonza) | • Heat inactivated FCS 10% v/v (Gibco^{™}) |
| | | • L-Glutamine 2mM (Gibco^{™}) |
| | | • Insulin 0.1% v/v (Sigma-Aldrich^{®}) |
| | | • EGF 0.02% v/v (Peprotech) |
| | | • Hydrocortisone 0.4% v/v (Sigma-Aldrich) |
| HUVEC | EBM-2 (Lonza) | • Hydrocortisone 0.4% v/v (Lonza) |
| | | • Human fibroblast derived growth factor-B (hFGF-B) 0.1% v/v (Lonza) |
| | | • Vascular endothelial growth factor (VEGF) 0.1% v/v (Lonza) |
| | | • R3-insulin derived growth factor-1 (R3-IGF-1) 0.1% v/v (Lonza) |
| | | • Ascorbic acid 0.1% v/v (Lonza) |
| | | • Human epidermal growth factor (hEGF) 0.1% v/v (Lonza) |
| | | • Foetal bovine serum 2% v/v (Lonza) |
| | | • Gentamycin and amphotericin (GA-1000) 0.1% v/v (Lonza) |

**Table 2 - Cell line information**

| **Cell source** | **Derivation, tumorigenicity** | **HPA labelling** | **Originator** |
|---|---|---|---|
| BT 474 | Derived from invasive ductal cancer, poorly tumorigenic in nude mice. Non metastatic. ER+, PR+/-, HER2+. Represents non-metastatic primary breast cancer (Valentiner et al., 2005; lorns et al., 2012) | + (Brooks et al., 2001) | Lasfargues et al. (1978) |
| MCF-7 | Derived from metastatic, malignant pleural effusion from invasive primary | ++++ (Brooks et al., 2001) | Soule et al. (1973) |
| | ductal breast cancer. Highly tumorigenic in animal models. ER+, PR+/-, HER2-. Represents metastatic breast cancer. (Schumacher and Adam, 1997; Valentiner et al., 2005) | | |
| SW480 | Derived from a primary adenocarcinoma of the colon, poorly tumorigenic in nude mice. Weakly metastatic. Represents non-metastatic colorectal cancer. (Hewitt et al., 2000) | - (Schnegelsberg et al., 2011) | Leibovitz et al. (1976) |
| HT29 | Derived from a primary adenocarcinoma of the colon, highly tumorigenic in animal models. Represents metastatic colorectal cancer (Schumacher et al., 1997) | ++++ (Lescar et al., 2007) | Fogh et al. (1975) |
| NCl-H322 | Derived from primary bronchioalveolar carcinoma of the lung, weakly tumorigenic in nude mice. Non-metastatic. Represents non-metastatic non-small-cell lung cancer (Mase et al., 2002) | - (Lescar et al., 2007) | Gazdar et al. (1990) |
| A549 | Derived from explant culture of lung carcinomatous tissue, highly tumorigenic in mouse models. Metastatic. Represents metastatic non-small cell lung cancer (Mase et al., 2002) | +/- (Lescar et al., 2007) | Giard et al., 1973 |
| BxPc-3 | Derived from primary adenocarcinoma of the pancreas. Weakly tumorigenic in animal models. Weakly metastatic. Represents non metastatic pancreatic cancer. (Suemizu et al., 2007) | +/- (McCater et al., 2013) | Tan et al. (1986) |
| MIA PaCa-2 | Derived from an undifferentiated cancer of the pancreas. Highly tumorigenic in mouse models. Metastatic. Represents metastatic pancreatic cancer (Suemizu et al., 2007) | ++ (McCarter et al., 2013) | Yunis et al. (1977) |

### Protein extraction and quantification from endothelial cells

HUVEC cells were grown in T175 flasks to confluence and then treated with 10ug/ml TNFα in complete growth medium for 2hrs at 37C in a humidified atmosphere. Cells were washed with ice-cold PBS. Cells were pelleted by brief centrifugation, supernatant was discarded and the pellet resuspended in 1x RIPA buffer with added 10ul/ml of protease inhibitor cocktail and placed on an end-over-end mixer at 4C for 30mins. After this time the tube was centrifuged at 13,000 RPM at 4°C for 20mins. The supernatant was transferred to a fresh 1.5ml tube and the pellet discarded. Extracted proteins were quantified using a BCA assay.

### SDS-PAGE and western blot

10ug of protein sample was used for SDS-PAGE with 3ul of Laemmli sample buffer and 1ul of 1M DTT. This was mixed and heated to 100C for 10min then placed on ice. The sample was run on a mini-PROTEAN TGX stain-free precast gel (12%) in 1x TGS buffer, with Precision Plus Protein Dual Colour Standard. The gel was run at 100 volts for 2hrs. The proteins were transferred by using a Trans-Blot Turbo Midi PVDF transfer pack. The gel was placed on the membrane and sandwiched between the layers, then placed in the Transblot Turbo machine on high MW setting. The membrane was washed in TBST (0.05% Tween) for 5min on a shaking platform, three times. The membrane was then blocked by incubating with 5% BSA/TBST for 2hrs on a shaking platform RT. Blocked membrane was incubated in 10ml of lug/ml GalNAc-BSA-Biotin in 5% BSA/TBST or 10ml of lug/ml BSA-Biotin in 5% BSA/TBST overnight at 4°C. Membranes were washed with 5% BSA/TBST for 5min three times, before incubating the membrane in 10ml of 5ug/ml streptavidin-HRP in 5% BSA-TBST at RT for 1 hour. The membrane was washed with 5% BSA-TBST for 5mins twice then once for 15mins. HRP substrate was prepared (Clarity + Clarity Max Western ECL Substrate, BioRad, 170-5060) in a 1:1 ratio and mixed. This was added to the membrane and imaged on a Chemidoc^{®} transilluminator.

### 2D PAGE

Activated total HUVEC protein at a concentration of 1ug/ul in 1x RIPA buffer with added protease inhibitors was cleaned-up for use in 2D PAGE by using 2D Clean-up kit from GE Healthcare, according to manufacturer's instructions (80-6484-51). The sample was prepared for 2D PAGE by using ReadyPrep 2-D starter kit from BioRad according to manufacturer's instructions (163-2105). This allowed the sample to be absorbed by the 11cm IPG strips and then subsequently separated by isoelectric-focusing in Ettan IPGphor 3 for 16hrs. The IPG strips were washed and rehydrated then placed in a Criterion^{™} XT Bis-Tris Precast Gels, 12% Gels were loaded into tanks and standard added and gels sealed with agarose. Agarose was allowed to set before filling tank with 1x MOPS buffer. 250ul of NuPage Antioxidant (Thermo, NP0005) was added per gel. Gels were run at 200 volts for 70 min. Gel to be blotted was removed from its cassette and washed twice in dH₂O. The remaining procedure was carried out at room temperature. The gel was placed in Invitrogen^{™} Novex^{™} SimplyBlue^{™} SafeStain (Thermo, LC6065) and placed on a shaking platform for 1hr. The gel was then de-stained in dH₂O overnight on a shaking platform. The gel was blotted using the iBlot^{™} 2 Dry Blotting System. Membrane was washed briefly in dH₂O and then blocked with 5% Marvel/TBST for 1hr on a rocking platform. The membrane was incubated with lug/ml GalNAc-BSA-Biotin in 5% BSA/TBST for 2 hr on a rocking platform and then washed 3 times for 10 min each with TBST on a rocking platform. The membrane was incubated with 5ug/ml Streptavidin-peroxidase in 5% BSA/TBST for 1hr on a rocking platform and then washed 3 times for 10 min each with TBST on a rocking platform. ECL HRP substrate was prepared and added to the membrane and then imaged using Chemidoc^{®} transilluminator.

### Preparation of gels for Mass Spectrometry

1D gels were stained with Coomassie blue and 2D gels were stained with silver stain and then bands and dots of interest were excised from the 1D and 2D gels using blots as a reference. Gels were then sent for mass spectrometry at Porton Biopharma Ltd.

### EV extraction from cancer cells

Cancer cells were grown in T175 flasks to 70% confluency and fed with 25ml/flask of EV cleared media (DMEM media containing 10% FBS, wherein the FBS has been previously spun for 16hrs at 120,000 g to clear existing EVs), and conditioned for 48hrs. Media was removed and spun at 300x g for 5 min then at 16,000x g for 20 mins at 4°C. Media was removed from flask to "normal" 50ml tube (Greiner Bio-One, 227285) and spun at 300x g for 5min. For microvesicles, transfer supernatant to high-speed (green or orange topped) 50 ml tube (Alpha Laboratories, CT1120), discard pellet, and centrifuge at 16000g for 20min. Transfer supernatant to normal 50ml tube - this is the EV portion. The pellet contains the microvesicles, which are pooled and resuspended in 20ml of PBS. This is then spun again at 16000g for 20mins. The supernatant is poured out of tube and the pellet resuspended in the volume of PBS that remains ~300ul. Block 0.22 micron (Fisher, fdr-050-071n) filter using 0.1% BSA (Sigma, A7906-100G) solution in PBS (40µl of 10% BSA in 40mls PBS) and filter media through this filter. Add media to Vivaspin 20 100 kDa concentrator (Fisher, 10774797). Concentrate the media by spinning at 3000g (swing bucket - 25 min) or 5000g (fixed angle rotor - 20 minute steps).

For exosomes, supernatant was filtered through a 0.22um filter which had been blocked with 0.1% BSA. The supernatant was concentrated using a Vivaspin 20, 100kDa concentrator to 500ul. SEC columns (BioRad, 7321010) were prepared by adding 14ml of sepharose and 10ml of PBS this was allowed to settle for 2hrs and then a column support was added and PBS allowed to flow through. The column was then washed three times with 10ml of PBS. 500ul of samples was added to the column support and allowed to absorb then 10ml of PBS added. 2.5ml of flow-through was collected and discarded then 2ml of flow-though was collected (containing EVs). EVs were quantified using Particlematrix^{™} particle analyser. EVs can then be stored in fridge for short term use.

### Endothelial static adhesion assay with or without EVs

Coverslips with activated endothelial cells were prepared as above. MCF7 breast cancer cells were grown in T75 flasks until 70% confluency and treated with 10mg/ml 8-hydroxypyrenetrisulphonic acid (HTPS) in complete medium for 2hrs at 37°C, 5% CO₂. After this time, HTPS was removed and cells were washed with 10ml of PBS 5 times until the wash ran clear. Cells were scraped and counted. 20,000 cells/ml was prepared. MCF7 cells were treated with either 500ul of EVs or 500ul of PBS and mixed for 10mins at RT. HUVEC cells were treated with either 500ul of EVs or 500ul of PBS at 37°C, 5% CO₂ for 30 mins. EVs or PBS was removed from cells and 20,000 cells/ml of MCF7 cells were added per well and incubated for 10mins, cells were removed and wells gently washed with warmed PBS to remove any unbound cells. Wells were then fixed with 4% PFA for 10mins at RT. Fixative was removed and the wells washed 3 times with PBS and then coverslips were mounted using fluoromount^{™}. Total adhered cells per coverslip were counted.

Coverslips with activated endothelial cells were prepared as above. MCF7 breast cancer cells were grown in T75 flasks until 70% confluency and treated with 10mg/ml HTPS in complete medium for 2hrs at 37C, 5% CO₂. HTPS was removed and cells were washed with 10ml of PBS 5 times until the wash ran clear. Cells were scraped and counted. 20,000 cells/ml was prepared. MCF7 cells were treated with either 0.1ug/ml recombinant plectin, (2b Scientific SKU RPC754Hu01 (residues Asp 175-Pro 400 of human plectin Uniprot ID Q15149, corresponding to the actin-binding domain), in PBS or PBS for 10mins at RT. Recombinant plectin or PBS were removed from cells and 20,000 cells/ml of MCF7 cells were added per well and incubated for 10mins, cells were removed and wells gently washed with warmed PBS to remove any unbound cells. Wells were then fixed with 4% PFA for 10mins at RT. Fixative was removed and the wells washed 3 times with PBS and then coverslips were mounted using fluoromount. Total adhered cells per coverslip were counted.

### Endothelial static adhesion assay with or without plectin silencing and EV rescue

MCF7 and HUVEC cells were grown in 24 well cell culture plates to 70% confluency the cells were treated with Dharmafect^{™} transfection reagent and 5uM of plectin siRNAs or scrambled negative control for 24hrs. Some HUVEC and MCF7 cells were non-treated. HUVEC cells were activated by treating with 10ug/ml TNFa in complete medium for 2hrs at 37°C, 5% CO₂. MCF7 breast cancer cells were grown in T75 flasks until 70% confluency and treated with 10mg/ml HTPS in complete medium for 2hrs at 37C, 5% CO₂. HTPS was removed and cells were washed with 10ml of PBS 5 times until the wash ran clear. Cells were scraped and counted. 20,000 cells/ml was prepared. MCF7 cells were treated with either 500ul of EVs isolated using the extraction method outlined above, or 500ul of PBS for 10mins at RT. EVs or PBS was removed and 1ml of MCF7 cells were added to HUVEC cells and incubated at 37C, 5% CO₂ for 10mins, before cells were removed and wells gently washed with warmed PBS to remove any unbound cells. Wells were then fixed with 4% PFA for 10mins at RT. Fixative was removed and the wells washed 3 times with PBS and then coverslips were mounted using fluoromount. Total adhered cells per coverslip were counted.

### Dot blot assays

Membrane isolation was done using plasma membrane protein extraction kit (Abcam, ab56400), exosomes/MV as in the attached protocol. Using a narrow-mouth pipette tip, spot 2 µl of sample or buffer onto the nitrocellulose membrane. Minimise the area that the solution penetrates (usually 3-4 mm) by applying it slowly. Let the membranes dry. ~30mins. Place each membrane inside a 6 well plate. Block by soaking membranes in 5% marvel in TBS-T or for HPA Carbo-free blocking solution (2BScientific, SP-5040-125) diluted in dH2O with 0.1% Tween 20 (1 hr, RT). Incubate with 10ug/ml biotinylated-HPA in Carbo-Block-T or 1:1,000 for all abs in 5% marvel in TBS-T at 37oC for 2hrs or 4oC overnight. Wash membranes three times with TBS-T (3x5min). Incubate with biotinylated anti-Ms (Vector Labs, BA-2000-1.5) or biotinylated anti-Rb (Vector Labs, BA-1000-1.5) in 5% marvel TBST at 37oC for 1hr. Wash membranes three times with TBS-T (3x5min). Incubated with lug/ml streptavidin-peroxidase in 5% marvel TBS-T or Carbo-Block-T for HPA. Wash three times with TBS (3x 10 min). Combine ECL substrates A and B (BioRad Clarity kit). Incubate each membrane with ECL reagent for 1 min, remove excessive solution from the surface, and image as a chemi-high sensitivity blot.

### HPA probing after Western Blot of MCF7 and BT474 breast cancer cells

MCF7 and BT474 cell lines were grown to 70% confluence in T75 flask and had their proteins extracted, quantified, run on SDS-PAGE and then blotted as previously described. Membrane was probed using biotinylated-HPA or anti-GAPDH antibody.

### Nanoview

Following standard protocol for R100 and analysis through Nanoviewer, EVs extracted from MCF7 and BT474 breast cancer cells using SEC (previously described) and labelled with fluorescently conjugated antibodies (CD81 and CD9) as well as HPA lectin.

### Preparation of activated endothelial cells on coverslips

Sterilised 13mm D glass coverslips were placed in wells of 24 well plate and 100,000 HUVEC cells were seeded. Cells were incubated at 37°C with 5% CO₂ atmosphere until cells became a monolayer. HUVEC cells were activated by treating with 10ug/ml TNFα in complete medium for 2hrs at 37°C, 5% CO₂.

### Endothelial adhesion assay with or without actin antibody inhibition

Coverslips with activated endothelial cells were prepared as above. MCF7 breast cancer cells were grown in T75 flasks until 70% confluency and treated with 10mg/ml HTPS in complete medium for 2hrs at 37°C, 5% CO₂. After this time HTPS was removed and cells were washed with 10ml of PBS 5 times until the wash ran clear. Cells were scraped and counted. 20,000 cells/ml was prepared. MCF7 cells were treated with either 1:1,000 actin IgG (Abcam ab8227), 1:1,000 GAPDH IgG or PBS for 30mins at RT. Antibodies or PBS were removed from cells and 20,000 cells/ml of MCF7 cells were added per well and incubated for 10mins, cells were removed and wells gently washed with warmed PBS to remove any unbound cells. Wells were then fixed with 4% PFA for 10mins RT. Fixative was removed and the wells washed 3 times with PBS and then coverslips were mounted using fluoromount. Total adhered cells per coverslip were counted.

### Flow cytometry

For the live cell studies, cultured cells were harvested around 70% confluence and detached from the bottom of the flask using Accutase. Cells were collected by centrifugation (1400rpm for 5mins) For analysis, cells with a concentration of 0.5x10⁶/ml were used. The cells were washed with PBS and incubated with HPA antibody for 10 minutes at RT in the dark (7.5µg/ml) in 3% BSA/TBS to detect HPA GalNAc epitope on the surface of the cells or 1/50 actin (clone SP124). Cells were treated with Propidium Iodide (10ug/ml) to gate live populations. The wavelength of the 647 conjugates was quantitatively recorded on the APC-A channel using a 13-color, 4-laser CytoFLEX S N-V-B-R Flow Cytometer, equipped with 375 nm, 405 nm, 488 nm and 638 nm lasers and operated using CytExpert Software.

Each sample was analysed in triplicate and determination of the GalNAc epitope levels on the surface of the analysed cells were based on the mean values of the fluorescence signals from the APC detector. Gating: 1) FSC-H vs FSC-A - To doublet discriminate, cells which have aggregated will have the double the area and height. 2) Pi Viability gate - To discriminate live cells from dead. Dead cells become permeabilized therefore PI will bind to nuclei therefore will have more florescence (I've also attached my unstained Pi control to show the differences) 3) Antibody/Lectin channel of interest to determine the shifts in peaks between different conditions.

For fixed cells, cells were harvested and resuspended in cold PBS. Cells were incubated them with anti-actin Ab for 1 h at 4°C (Proteintech cat: 60008-1-Ig). Cells were then washed and resuspended in PBS before flow cytometry.

### NanoFCM

NanoFCM is a 40nm to 1 micron flow cytometer that focuses on exosome analysis as independently reviewed in https://onlinelibrary.wiley.com/doi/epdf/l 0.1002/jev2.12044 and https://doi.org/10.1002/jev2.12079. All of these results were run by the company based in Nottingham, UK. Exosomes were extracted as previously explained. The machine was set with the following settings: SN-N30E, V1.11 software, sample pressure-1 Kpa, laser-10/50 mW 488@ 20/100 mW 640, SS decay -10% and Min width - 0.3ms. Calibrated with silica nanoparticles and EVs concentration quantified. PBS, antibody controls and EV unstained and stained optimisation carried out. For actin antibodies, approx 2e8 to 2e10 particles, incubated with antibody/stain at 1in50 to 1in500 for 30 mins at room temperature and diluted in PBS to 1in5 to 1in50. For HPA, approx 2e8 particles were incubated with antibody/stain at 1in500 for 30 mins at room temperature, then diluted 1in20 to 1in50 in PBS. If high background, then ultra-centrifugated at 110k xg for 1 hour and resuspended to 50µl. Supernatant was removed and samples were resuspended in 50ul PBS. Events gated for between approx 40 - 199nm recorded for 1 min and recorded as number of exosomes with a positive signal as a percentage of total exosomes gated at that size.

**Table 3 -Antibodies used**

| **Ab Name** | **Reason for using** | **Manufacture** | **Item Code** | **Host Species** | **For WB use at** |
|---|---|---|---|---|---|
| EpCAM | Cell surface specific marker | Abcam | ab71916 | Rb | 1ug/ml |
| GAPDH | Cytosolic specific marker | Abcam | ab128915 | Rb | 1/10,000 - 1/50,000 |
| IgG | Isotype control to check specificity of ab | Abcam | ab172730 | Rb | - |
| ACTB 1 | | Proteintech | 66009-1-IG | Ms | 1/5,000 - 1/50,000 |
| ACTB 2 | | Abcam | ab8227 | Rb | 1/1,000 - 1/5,000 |
| ACTB 3 | | Abcam | ab8226 | Ms | 1ug/ml |
| ACTB 4 | | Invitrogen | MA1-140 | Ms | 1/5,000-1/20,000 |
| ACTG 1 | | BioRad | VMA00049 | Ms | 1/1,000 |
| ACTG 2 | | Sigma | A8481 | Ms | 0.25-0.5ug/ml |
| ACTBF1 | Ab used for flow, to check working | Abcam | ab206277 | Rb | - |
| ACTBF2 | Ab used for flow | Abcam | ab267562 | Rb | - |
| ACTBFITC | Ab used for NanoFCM | Abcam | ab6277 | Ms | - |
| ACTGFITC | Ab used for NanoFCM | Santa Cruz Biotechnology | sc-65638 FITC | Ms | - |
| HPA | | Sigma | L6512-1MG | - | - |
| CD81* | Positive EV control | Abcam | ab79559 | Ms | 2ug/ml |
| TSG101* | Positive EV control | Abcam | ab30871 | Rb | 1ug/ml |
| Hsp70* | Positive EV control(?) | Abcam | ab5439 | Ms | 1/1,000 |
| GM130* | Negative EV control | Abcam | ab52649 | Rb | 1/1,000 - 1/10,000 |
| CD9* | Positive EV control | Cambridge Bioscience | EXOAB-CD9A-1 | Rb | - |
| CD63* | Positive EV control | Novus Biologicals | NBP2-42225 | Ms | 2ug/ml |
| Cytochrome C* | Negative EV control | Abcam | ab150422 | Rb | 1/1,000 - 1/10,000 |

### EXAMPLES

### Example 1 - identification of membrane-bound actin as a biomarker for metastatic potential

Binding of Helix pomatia agglutinin (HPA), a lectin from the Roman snail, to breast cancer (BC) and other epithelial cancers is associated with metastatic competence and poor patient prognosis (Brooks S., 2000, Histology and Histopathology, 15(1): 143-158). What HPA binds to specifically is not known, however, it has been shown that HPA binds to a range of glycoproteins with terminal α-GalNAc (the monosaccharide with which HPA has then highest affinity). One of these glycoproteins is the cancer associated Tn antigen (Ser/Thr-GalNAc) which is the initial structure made in O-linked glycosylation which is normally always further elaborated, but is a common occurrence in cancer (Brooks S.A. & Leathem A.J.C., 1995, British J. Cancer, 71: 1033-1038).

Highly HPA-positive BC cells adhere significantly more to endothelial cells in a static adhesion assay than weakly HPA-positive or HPA-negative BC cells. This assay mimics metastatic cancer cell arrest in narrow capillaries. For cancer cells to successfully metastasise they must bind to and extravasate out of the blood vessel. It has been shown that binding is mediated through this glycosylation mark as incubating cells with HPA or BSA-GalNAc inhibits cells binding.

### Identification of the receptor/s on the endothelial cells which mediate HPA-positive cell adhesion.

In order to identify the putative receptor/s on endothelial cells which are recognising the HPA-binding glycans on the BC cells, total protein was extracted from the endothelial cells that had been activated using TNFα (an inflammatory cytokine known to upregulate endothelial receptors). Protein was run on SDS-PAGE and then blotted. GalNAc-BSA- biotin (GalNAc is the sugar with which HPA has the greatest nominal specificity for) was used as a probe to identify endothelial proteins of interest. As a negative control BSA-biotin was also used to probe the membrane. As illustrated in Figure 1, when the membrane was probed with GalNAc-BSA-biotin several bands were observed, whilst no bands were observed when the membrane was probed with BSA-biotin.

Several bands were consistently identified using GalNAc-BSA-biotin as a probe, from these, five gel fragments were selected and excised for mass spec (Figure 2). Bands observed in blot from 1D gel could represent a large number of proteins as separation is only on size. To better resolve the bands, 2D-PAGE was used to separate proteins on both size and pH. 2D gels were blotted and probed with GalNAc-BSA-Biotin. Thirteen spots were identified as of interest and were excised from the 2D PAGE gel (Figure 3).

Mass spec data revealed several proteins in the gel fragments from both 1D and 2D-PAGE, some of which overlapped in both. Amongst those proteins identified was plectin, which is normally an intracellular scaffold protein. However, Shin et al. (2013) demonstrated that plectin can localise to the membrane of cells through exosome secretion. To investigate this, EVs were isolated from BC cells and incubated with either BC cells, endothelial cells or both prior to the static adhesion assay. It was found that incubating both the BC cells and endothelial cells with EVs significantly increased binding of BC cells to endothelial cells when compared to non-treated, BC cell only or endothelial cell only treated (Figure 4). This finding suggested that EVs may have a positive role in aiding BC cell adhesion to endothelial cells.

To investigate this further, plectin was knocked down in either the BC cells, endothelial cells or both, using siRNA. In all instances, binding of BC cells to endothelial cells was significantly reduced when compared to both non-treated and scrambled siRNA treated cells (termed neg on graph) negative control cells, therefore suggesting that plectin is needed for BC cells to bind to endothelial cells (Figure 5).

### Confirmation that the receptor on the endothelial cells is specific for the glycosylation marks on the BC cells and mediates cell binding

To confirm that the receptor on the endothelial cells is specific for the glycosylation mark on the BC cells, a recombinant plectin fragment (containing the actin-binding domain of plectin, which can bind all actin isoforms (Fontao et al., 2001, J. Cell. Sci., 114: 2065-2076)) was used to block BC cells, by incubating for 10 mins prior to addition of the endothelial cells. Cell adhesion to the endothelial cells was significantly reduced when compared to non-treated control cells (Figure 6). It was hypothesized that the reduced binding observed when incubating the BC cells with the recombinant plectin is a result of the plectin binding to the HPA-positive glycosylation mark, thereby "capping" them and preventing binding to the endothelial cells.

### Investigation of how EVs from BC cells increase adhesion to endothelial cells

The findings that knockdown of plectin in the BC cells, combined with the observation that EVs from BC cells can increase the adhesion of BC cells to endothelial cells, led to the hypothesis that EVs can carry plectin from BC cells to the surface of endothelial cells, which then increase the effective "landing platform" for BC cells to bind to. To begin testing this hypothesis, a "rescue" experiment was performed. Binding of BC cells to endothelial cells was reduced when plectin was knocked down in BC cells (Figure 7). Interestingly, this reduced binding was partially rescued when endothelial cells were treated with EVs from control BC cells. This is consistent with the hypothesis that the EVs from the normal cells (which have plectin) are providing the "landing platform" to allow binding to occur.

In a model system, two BC cell lines were used which have previously been reported to be highly HPA positive (MCF7) and weakly HPA positive (BT474) to verify the above hypothesis. Protein was extracted from the two cell lines and run on SDS-PAGE then blotted and probed with HPA. The HPA-positive BC cell line (MCF7) possessed a greater range of HPA positive glycoproteins than the weakly HPA-positive BC cell line (BT474), which has only one band at 75 kDa, thereby confirming the use of this model system (Figure 8).

Using NanoView, it was shown that HPA-binding-positive cells produce HPA-binding-positive EVs and likewise HPA-binding-negative cells produce HPA-binding-negative EVs. To do this, EVs were isolated from two BC cell lines, one HPA-binding-negative and the other HPA-binding-positive, by labelling the two EV populations with fluorescently conjugated antibodies specific for two commonly used EV markers and HPA it was possible to quantify the amount the EVs had 'HPA-binding-positivity'. EVs from HPA-binding-negative BC cells (BT474 left, Figure 9) were found to be HPA-binding-negative whist EVs from HPA-binding-positive BC cells (MCF7 right, Figure 9) were HPA-binding-positive. In particular, HPA was predominately colocalized with CD9 on vesicles - a maximum of 45% of CD9 positive vesicles are HPA positive compared to 13% of CD81 positive and 9% of CD63 positive vesicles. Due to overlap with the APC emission spectrum, fluorescently conjugated anti-CD63 was not included in this experiment.

### Identification of the glycoprotein on BC cells which mediates binding to the receptor on the endothelial cells

Cytoskeletal plectin has a number of binding partners which interact with its actin-binding domain (ABD), including beta-actin. To investigate whether glycosylated beta-actin is a potential binding partner of cell surface plectin, a blocking experiment was performed, where beta-actin was masked using an anti beta-actin IgG (Abcam ab8227) for 30mins prior to incubation with endothelial cells). Compared to untreated control cells, when beta-actin is blocked, cell adhesion is significantly reduced, therefore suggesting that beta-actin is a binding-partner for plectin and that this interaction at contributes to the binding of BC cells to endothelial cells (Figure 10).

### Levels of surface actin on different cell lines

A range of cells with known invasiveness and HPA-binding profiles were used to determine the level of surface actin on different cell types by flow cytometry. These ranged from normal/HPA-negative, to non-metastatic/weakly HPA-positive to highly metastatic/highly HPA-positive (Figure 11). HME cells are normal breast cells and are HPA-negative; BT474 is derived from a primary invasive ductal cancer, with no evidence of metastases (Lasfargues et al. 1978, J. Nat. Cancer Institute, 61(4): 967-978) and labels weakly with HPA; ZR 751 was derived from malignant ascites from an invasive primary ductal breast cancer - i.e. from cells that had already metastasised (Engel et al. 1978, Canc. Res., 38, 3352-3364) and labels moderately with HPA; T47D was derived from malignant pleural effusion from an invasive primary ductal breast cancer - i.e. from cells that had already metastasised (Keydar et al 1979, Eur J Cancer, 15(5):659-70) and labels highly with HPA; MCF7 was derived from malignant pleural effusion from an invasive primary ductal breast cancer - i.e. from cells that had already metastasised (Soule et al. 1973, J. Nat. Cancer Institute, 51(5): 1409-14-16) and labels highly with HPA. Actin labelling appears to follow a similar trend as HPA labelling, in that increased surface actin is measured in more invasive/ metastatic cells.

Figure 12 further demonstrates, using flow cytometry, that the level of actin is greater on the surface of metastatic breast cancer cells (MCF7) than non-metastatic breast cancer cells (BT474). This data was generated using an antibody capable of detecting both beta and gamma actin. Figure 13 further demonstrates that metastatic cancers, including breast MCF7, colorectal HT-29, and lung A539, also display elevated levels of HPA on their surface. These results further demonstrate that surface levels of both actin and HPA are increased in metastatic cancer cells, thus demonstrating their diagnostic potential.

Figure 14 further supports the increased surface levels of actin on metastatic cancer cells, this data compares non metastatic and metastatic breast cancer and uses antibodies to either beta actin or gamma actin, both of which show increased actin levels on the metastatic cancer cells.

### Levels of surface actin on microvesicles obtained from different cell lines

Figures 15 to 17 compare the level of actin on the surface of microvesicles obtained from metastatic and non-metastatic breast, lung and pancreatic cancer cells. The results clearly show elevated levels of actin on microvesicles obtained from the metastatic cells.

### Levels of surface actin on exosomes obtained from different cell lines

Figures 18 to 20 compare the level of actin on the surface of exosomes obtained from metastatic and non-metastatic breast and colorectal cancer cells. The results clearly show elevated levels of actin on exosomes obtained from the metastatic cells when compared to the non-metastatic cells. Figure 21 further demonstrates that exosomes obtained from metastatic cancers, specifically colorectal HT-29 cancer cells, also display elevated levels of HPA on their surface. Figure 22 shows that metastatic breast and colorectal cancer cells display elevated levels of gamma actin on their surface.

### Discussion

In conclusion, the data presented herein demonstrates that actin on the surface (membrane-bound) of epithelial cancer cells, and/or extracellular vesicles derived therefrom, can serve as a diagnostic or prognostic marker of the metastatic potential of the cancer cells. The actin levels may also be used to monitor response to a specific cancer treatment, to monitor disease progression and/or to monitor disease reoccurrence, HPA levels may also be used as a biomarker, either alone or in combination with actin levels.

## Claims

1. A method of determining whether a subject who has been diagnosed with cancer is likely to develop, or is likely to have developed, a metastasis, wherein the method comprises:
a. measuring the level of membrane-bound actin in a sample obtained from the subject,
b. comparing the level of membrane-bound actin measured in the sample obtained from the subject with the level of membrane-bound actin in a reference sample, and
c. determining that the subject is likely to develop metastasis or is likely to have developed metastasis if the level of membrane-bound actin in the sample obtained from the subject is higher than the level of membrane-bound actin in the reference sample.

2. A method of diagnosing a subject with metastatic cancer, wherein the method comprises:
a. measuring the level of membrane-bound actin in a sample obtained from the subject,
b. comparing the level of membrane-bound actin measured in the sample obtained from the subject with the level of membrane-bound actin in a reference sample, and
c. diagnosing the subject with metastatic cancer when the level of membrane-bound actin in the sample obtained from the subject is higher than the level of membrane-bound actin in the reference sample.

3. A method of prognosing a subject who has been diagnosed with cancer, wherein the method comprises:
a. measuring the level of membrane-bound actin in a sample obtained from the subject,
b. comparing the level of membrane-bound actin measured in the sample with the level of membrane-bound actin in a reference sample,
c. determining that the patient has a poor prognosis when the level of membrane-bound actin in the sample obtained from the subject is higher than the level of membrane-bound actin in the reference sample.

4. The method of claim 3, wherein poor prognosis relates to an increased likelihood of developing a metastasis and/or a reduced chance of survival.

5. A method of identifying a patient who has been diagnosed with cancer who is likely to benefit from treatment with a known treatment for metastatic cancer, wherein the method comprises:
a. measuring the level of membrane-bound actin in a sample obtained from the subject,
b. comparing the level of membrane-bound actin measured in the sample with the level of membrane-bound actin in a reference sample,
c. determining that the patient is likely to benefit from treatment with a known treatment for metastatic cancer if the level of membrane-bound actin in the sample obtained from the subject is higher than the level of membrane-bound actin in the reference sample.

6. The method of any of claims 1-5, wherein the level of membrane-bound actin in the sample obtained from the subject is determined to be higher than the level of membrane-bound actin in the reference sample when it is at least about 50%, 75%, 100%, 150%, 200%, 300%, 500%, 1000%, 5000%, or 10000% more than the level of membrane-bound actin in the reference sample.

7. The method of any of claims 1-6, wherein the reference sample is an equivalent sample from a healthy patient who has not been diagnosed with cancer, or an equivalent sample which is known to be metastatic.

8. The method of any of claims 1-7, wherein the method is an *ex vivo* or *in vitro* method.

9. The method of any of claims 1-8, wherein the sample is a tissue biopsy and/or a blood sample and/or a saliva sample.

10. The method of claim 9, wherein the tissue biopsy is a breast tissue biopsy.

11. The method of any of claims 1-10, wherein detecting or measuring the level of membrane-bound actin is performed by flow cytometry, immunohistochemistry, immunocytochemistry, western blot, and/or ELISA.

12. The method of any of claims 1-11, wherein if:
a. it is determined that that subject is likely to develop or is likely to have developed a metastasis, or
b. the subject is diagnosed with metastatic cancer, or
c. the subject is given a poor prognosis, or
d. it is determined that the subject is likely to benefit from treatment for metastatic cancer, then
a therapeutic for metastatic cancer is administered to the subject.

13. The method of any of claims 1-12, wherein the membrane bound actin is detected in the membrane of a cell, and/or of an extracellular vesicle; optionally
wherein the cell is an epithelial cell or a circulating tumour cell; optionally
wherein the epithelial cell is from breast tissue.

14. The method of any of claims 1-13, wherein the cancer is an epithelial cell cancer; optionally wherein the epithelial cell cancer is breast cancer.

15. The method of any of claims 1-14, wherein the actin is beta-actin or gamma-actin.

## Patentansprüche

1. Verfahren zum Bestimmen, ob es bei einem Subjekt, bei dem Krebs diagnostiziert wurde, wahrscheinlich ist, dass es eine Metastase entwickelt, oder es wahrscheinlich ist, dass sich diese bereits entwickelt hat, wobei das Verfahren Folgendes umfasst:
a. Messen des Spiegels an membrangebundenem Aktin in einer Probe, die von dem Subjekt erlangt wird,
b. Vergleichen des Spiegels an membrangebundenem Aktin, der in der von dem Subjekt erlangten Probe gemessen wurde, mit dem Spiegel an membrangebundenem Aktin in einer Referenzprobe und
c. Bestimmen, dass es wahrscheinlich ist, dass das Subjekt eine Metastase entwickelt, oder es wahrscheinlich ist, dass es diese bereits entwickelt hat, wenn der Spiegel an membrangebundenem Aktin in der von dem Subjekt erlangten Probe höher als der Spiegel an membrangebundenem Aktin in der Referenzprobe ist.

2. Verfahren zum Diagnostizieren von metastatischem Krebs bei einem Subjekt, wobei das Verfahren Folgendes umfasst:
a. Messen des Spiegels an membrangebundenem Aktin in einer Probe, die von dem Subjekt erlangt wird,
b. Vergleichen des Spiegels an membrangebundenem Aktin, der in der von dem Subjekt erlangten Probe gemessen wurde, mit dem Spiegel an membrangebundenem Aktin in einer Referenzprobe und
c. Diagnostizieren von metastatischem Krebs bei dem Subjekt, wenn der Spiegel an membrangebundenem Aktin in der von dem Subjekt erlangten Probe höher als der Spiegel an membrangebundenem Aktin in der Referenzprobe ist.

3. Verfahren zur Prognose für ein Subjekt, bei dem Krebs diagnostiziert wurde, wobei das Verfahren Folgendes umfasst:
a. Messen des Spiegels an membrangebundenem Aktin in einer Probe, die von dem Subjekt erlangt wird,
b. Vergleichen des Spiegels an membrangebundenem Aktin, der in der Probe gemessen wurde, mit dem Spiegel an membrangebundenem Aktin in einer Referenzprobe und
c. Bestimmen, dass der Patient eine schlechte Prognose hat, wenn der Spiegel an membrangebundenem Aktin in der von dem Subjekt erlangten Probe höher als der Spiegel an membrangebundenem Aktin in der Referenzprobe ist.

4. Verfahren nach Anspruch 3, wobei sich die schlechte Prognose auf eine erhöhte Wahrscheinlichkeit für die Entwicklung einer Metastase und/oder eine reduzierte Überlebenswahrscheinlichkeit bezieht.

5. Verfahren zum Identifizieren eines Patienten, bei dem Krebs diagnostiziert wurde und bei dem es wahrscheinlich ist, dass er von einer Behandlung mit einer bekannten Behandlung für metastatischen Krebs profitiert, wobei das Verfahren Folgendes umfasst:
a. Messen des Spiegels an membrangebundenem Aktin in einer Probe, die von dem Subjekt erlangt wird,
b. Vergleichen des Spiegels an membrangebundenem Aktin, der in der Probe gemessen wurde, mit dem Spiegel an membrangebundenem Aktin in einer Referenzprobe und
c. Bestimmen, dass es wahrscheinlich ist, dass der Patient von einer Behandlung mit einer bekannten Behandlung für metastatischen Krebs profitiert, wenn der Spiegel an membrangebundenem Aktin in der von dem Subjekt erlangten Probe höher als der Spiegel an membrangebundenem Aktin in der Referenzprobe ist.

6. Verfahren nach einem der Ansprüche 1-5, wobei bestimmt wird, dass der Spiegel an membrangebundenem Aktin in der von dem Subjekt erlangten Probe höher als der Spiegel an membrangebundenem Aktin in der Referenzprobe ist, wenn er mindestens etwa 50 %, 75 %, 100 %, 150 %, 200 %, 300 %, 500 %, 1000 %, 5000 % oder 10.000 % höher als der Spiegel an membrangebundenem Aktin in der Referenzprobe ist.

7. Verfahren nach einem der Ansprüche 1-6, wobei die Referenzprobe eine gleichwertige Probe von einem gesunden Patienten ist, bei dem kein Krebs diagnostiziert wurde, oder eine gleichwertige Probe ist, von der bekannt ist, dass sie metastatisch ist.

8. Verfahren nach einem der Ansprüche 1-7, wobei das Verfahren ein Ex-vivo-Verfahren oder ein In-vitro-Verfahren ist.

9. Verfahren nach einem der Ansprüche 1-8, wobei die Probe eine Gewebebiopsie und/oder eine Blutprobe und/oder eine Speichelprobe ist.

10. Verfahren nach Anspruch 9, wobei die Gewebebiopsie eine Brustgewebebiopsie ist.

11. Verfahren nach einem der Ansprüche 1-10, wobei das Detektieren oder Messen des Spiegels an membrangebundenem Aktin durch Durchflusszytometrie, Immunhistochemie, Immunozytochemie, Western-Blot und/oder ELISA durchgeführt wird.

12. Verfahren nach einem der Ansprüche 1-11, wobei, wenn:
a. bestimmt wird, dass es wahrscheinlich ist, dass das Subjekt eine Metastase entwickelt, oder es wahrscheinlich ist, dass es diese bereits entwickelt hat, oder
b. bei dem Subjekt metastatischer Krebs diagnostiziert wird oder
c. dem Subjekt eine schlechte Prognose ausgestellt wird oder
d. bestimmt wird, dass es wahrscheinlich ist, dass das Subjekt von einer Behandlung für metastatischen Krebs profitiert, dann dem Subjekt eine Therapeutikum für metastatischen Krebs verabreicht wird.

13. Verfahren nach einem der Ansprüche 1-12, wobei das membrangebundene Aktin in der Membran einer Zelle und/oder eines extrazellulären Vesikels detektiert wird; optional
wobei die Zelle eine Epithelzelle oder eine zirkulierende Tumorzelle ist; optional
wobei die Epithelzelle aus Brustgewebe stammt.

14. Verfahren nach einem der Ansprüche 1-13, wobei der Krebs Epithelzellenkrebs ist; optional wobei der Epithelzellenkrebs Brustkrebs ist.

15. Verfahren nach einem der Ansprüche 1-14, wobei das Aktin Beta-Aktin oder Gamma-Aktin ist.

## Revendications

1. Procédé permettant de déterminer si un sujet chez qui un cancer est diagnostiqué est susceptible de développer, ou est susceptible d'avoir développé, une métastase, le procédé comprenant les étapes consistant à :
a. mesurer le niveau d'actine membranaire dans un échantillon obtenu auprès du sujet,
b. comparer le niveau d'actine membranaire mesuré dans l'échantillon obtenu du sujet avec le niveau d'actine membranaire dans un échantillon de référence, et
c. déterminer que le sujet est susceptible de développer une métastase ou est susceptible d'avoir développé une métastase si le niveau d'actine membranaire dans l'échantillon obtenu du sujet est supérieur au niveau d'actine membranaire dans l'échantillon de référence.

2. Procédé de diagnostic d'un sujet atteint d'un cancer métastatique, le procédé comprenant les étapes consistant à :
a. mesurer le niveau d'actine membranaire dans un échantillon obtenu auprès du sujet,
b. comparer le niveau d'actine membranaire mesuré dans l'échantillon obtenu du sujet avec le niveau d'actine membranaire dans un échantillon de référence, et
c. diagnostiquer un cancer métastatique chez le sujet lorsque le niveau d'actine membranaire dans l'échantillon obtenu du sujet est supérieur au niveau d'actine membranaire dans l'échantillon de référence.

3. Procédé de pronostic d'un sujet chez qui un cancer est diagnostiqué, le procédé comprenant les étapes consistant à :
a. mesurer le niveau d'actine membranaire dans un échantillon obtenu auprès du sujet,
b. comparer le niveau d'actine membranaire mesuré dans l'échantillon avec le niveau d'actine membranaire dans un échantillon de référence,
c. déterminer que le pronostic du patient est mauvais lorsque le niveau d'actine membranaire dans l'échantillon obtenu du sujet est supérieur au niveau d'actine membranaire dans l'échantillon de référence.

4. Procédé selon la revendication 3, dans lequel un mauvais pronostic est lié à une probabilité accrue de développer une métastase et/ou à une chance réduite de survie.

5. Procédé d'identification d'un patient chez qui un cancer a été diagnostiqué et qui est susceptible de bénéficier d'un traitement avec un traitement connu contre le cancer métastatique, le procédé comprenant les étapes consistant à :
a. mesurer le niveau d'actine membranaire dans un échantillon obtenu auprès du sujet,
b. comparer le niveau d'actine membranaire mesuré dans l'échantillon avec le niveau d'actine membranaire dans un échantillon de référence,
c. déterminer que le patient est susceptible de bénéficier d'un traitement avec un traitement connu contre le cancer métastatique si le niveau d'actine membranaire dans l'échantillon obtenu du sujet est supérieur au niveau d'actine membranaire dans l'échantillon de référence.

6. Procédé selon l'une quelconque des revendications 1 à 5, dans lequel le niveau d'actine membranaire dans l'échantillon obtenu à partir du sujet est déterminé comme étant supérieur au niveau d'actine membranaire dans l'échantillon de référence lorsqu'il est au moins environ 50 %, 75 %, 100 %, 150 %, 200 %, 300 %, 500 %, 1000 %, 5000 % ou 10000 % supérieur au niveau d'actine membranaire dans l'échantillon de référence.

7. Procédé selon l'une quelconque des revendications 1 à 6, dans lequel l'échantillon de référence est un échantillon équivalent provenant d'un patient en bonne santé qui n'a pas reçu de diagnostic de cancer, ou un échantillon équivalent qui est connu pour être métastatique.

8. Procédé selon l'une quelconque des revendications 1 à 7, dans lequel le procédé est un procédé *ex vivo* ou *in vitro.*

9. Procédé selon l'une quelconque des revendications 1 à 8, dans lequel l'échantillon est une biopsie tissulaire et/ou un échantillon de sang et/ou un échantillon de salive.

10. Procédé selon la revendication 9, dans lequel la biopsie tissulaire est une biopsie de tissu mammaire.

11. Procédé selon l'une quelconque des revendications 1 à 10, dans lequel la détection ou la mesure du niveau d'actine membranaire est effectuée par cytométrie de flux, immunohistochimie, immunocytochimie, western blot et/ou ELISA.

12. Procédé selon l'une quelconque des revendications 1 à 11, dans lequel si :
a. il est déterminé que le sujet est susceptible de développer ou est susceptible d'avoir développé une métastase, ou
b. le sujet reçoit un diagnostic de cancer métastatique, ou
c. le sujet reçoit un mauvais pronostic, ou
d. il est déterminé que le sujet est susceptible de bénéficier d'un traitement contre le cancer métastatique, alors
un traitement contre le cancer métastatique est administré au sujet.

13. Procédé selon l'une quelconque des revendications 1 à 12, dans lequel l'actine membranaire est détectée dans la membrane d'une cellule et/ou d'une vésicule extracellulaire ; éventuellement
dans lequel la cellule est une cellule épithéliale ou une cellule tumorale circulante ; éventuellement
dans lequel la cellule épithéliale provient du tissu mammaire.

14. Procédé selon l'une quelconque des revendications 1 à 13, dans lequel le cancer est un cancer des cellules épithéliales ; éventuellement dans lequel le cancer des cellules épithéliales est un cancer du sein.

15. Procédé selon l'une quelconque des revendications 1 à 14, dans lequel l'actine est la bêta-actine ou la gamma-actine.
